# EUROPEAN PATENT APPLICATION

(11) **EP 1 815 864 A1**
(43) Date of publication of application: **08.08.2007**
(21) Application number: 05806933.7
(22) Date of filing: 15.11.2005
(51) Int. Cl.: A61K 39/395, A61P 35/04

(54) **METHOD OF SUPPRESSING METASTASIS OF CANCER CELLS AND MEDICINAL COMPOSITION TO BE USED THEREFOR**

(30) Priority: 15.11.2004 JP 2004330814
(71) Applicant: KIRIN BEER KABUSHIKI KAISHA, Tokyo 104-8288 (JP); CENTRAL INSTITUTE FOR EXPERIMENTAL ANIMALS, Kawasaki-shi, Kanagawa 216-0001 (JP)
(72) Inventor: TOKORO, Yusuke, c/o Kirin Beer Kabushiki Kaisha, Takasaki-shi, Gunma 370-1295 (JP); NISHIKAWA, Mitsuo,, Takasaki-shi, Gunma 370-0828 (JP)
(74) Representative: HOFFMANN EITLE
(86) International application number: PCT/JP2005/020950
(87) International publication number: WO 2006/051984

(57) **Abstract**

The present invention provides a novel method for inhibiting cancer cell metastasis using an antibody against TM7XN1, a pharmaceutical composition to be used for the method, and a cancer-metastasis-suppressing agent comprising an antibody against TM7XN1 as an active ingredient.

## Description

### Technical Field

The present invention relates to a novel method for suppressing metastasis of various cancer cells expressing TM7XN1 through the use of an antibody against a specific cell surface receptor referred to as TM7XN1 and a pharmaceutical composition to be used for the method.

### Background Art

### Cancer metastasis

Even after complete removal of a primary cancer, a malignant tumor often metastasizes. A metastatic malignant tumor is formed at a location distant from the primary lesion as a result of the metastasis of the primary tumor. This is one of the most important problems in cancer therapy. Specifically, even if a primary lesion is treated, a patient will die because of the growth of a tumor that has metastasized to another organ. Accordingly, malignant tumor metastasis is the most common reason for failed cancer therapies (see Bertino et al., (edited in 1996), Encyclopedia of Cancer, Academic Press; Devita et al., (edited in 1997), Cancer: Principles & Practice of Oncology, Lippincott, Williams and Wilkins; Cavalli et al., (1996), Textbook of Medical Oncology, Dunitz Martin Ltd; Peckham et al., (edited in 1995), Oxford Textbook of Oncology, Oxford Univ. Press; and Mendelsohn et al., (1995), The Molecular Basis of Cancer, Saunders, Philadelphia).

For example, in the case of many types of clinically diagnosed solid cancer (a type of tumor that is a primary lesion resulting from the local growth of cancer), surgical obliteration is thought to be the first means for treatment. However, primary cancer cell metastasis is often observed after surgical operation. Cancer infiltration at a metastatic site spreads over the whole body, so that the patient will die due to the growth of metastatic cancer. It has been reported that for individual bodies having resectable tumors, primary tumor growth or local recurrence are often causes of death. It is currently considered that almost 40% of cancer victims with operable tumors will finally die because of metastatic disease following surgical operation.

Examples of currently available cancer treatment methods include surgical therapy, radiotherapy, chemotherapy, and immunological methods. When cancer cannot be treated by surgical therapy, chemotherapy or radiation therapy is applied. However, a chemotherapeutic agent blocks the growth of cancer cells via cell growth inhibition. Chemotherapy is broadly used for cancer treatment; however, it is a mode of non-specific treatment that affects not only growing cancer cells, but also actively growing normal cells. Hence, chemotherapy often causes severe side effects (e.g., immune suppression, pancytopenia (anemia, thrombocytopenia, and bone marrow cell growth inhibition accompanied by leucopenia), diarrhea, vomiting, and epilation (hair reduction)). Radiotherapy is also a mode of non-specific treatment that blocks the growth of cancer cells via cell growth inhibition. Hence, radiotherapy has problems similar to those in the case of chemotherapy. On the other hand, it is considered that the possibility of immunotherapy to cause severe side effects is lower than those in the case of chemotherapy or radiation therapy. However, immunotherapy exerting a strong effect of blocking cancer growth has not yet been established.

Cancer therapy is performed in a multidisciplinary manner through combination of the above therapeutic methods. However, it is known to have very low effectiveness against recurrent cancer. Furthermore, these therapeutic methods are also known to have almost no effects against existing metastatic tumors. Moreover, all of the above methods are also known to have a limited metastasis-suppressing effect. Accordingly, it is clear that a drug exerting a metastasis-suppressing effect can be one of the means to solve the problems of current cancer therapy.

Malignant melanoma, breast cancer, lung cancer, colon cancer, and prostate cancer are thought to be cancer types that tend to metastasize. The range of metastasis differs depending on a cancer type. The lungs and the liver are well known as target organs of cancer, and the brain or the bone marrow is also a target organ at a high frequency. Bone metastasis differs from metastasis to other organs, such that it rarely directly threatens life. However, bone metastasis is complicated by excruciating bone ache, the restriction of physical activity, or the like, thereby significantly lowering patient QOL and indirectly causing one's early death.

Cancer cell metastasis takes place when cancer cells enter a blood vessel from the primary lesion, reach a target organ with circulating blood stream, come out of the blood vessel at the site, and then adhere and grow at the organ. This process is common among metastasis to the lungs, metastasis to the liver, metastasis to the brain, metastasis to the bone marrow, and the like. However, establishment of metastasis to the bone marrow requires two processes: a process during which cancer cells enter the bone marrow from a nutrient vessel on the periosteum via the venous sinus; and a final process during which the cancer cells adhere and grow on the surface of the endosteum.

Based on such cancer metastasis mechanism, development of various metastasis-suppressing agents has been attempted, such as a drug for blocking cancer from entering a blood vessel or a drug for blocking cancer from entering a target organ from the blood vessel. A method that enables effective suppression of cancer cell metastasis has not yet been established. Furthermore, a drug having an effect of suppressing cancer cell metastasis has not yet been marketed.

As described above, a method for efficiently inhibiting tumor metastasis, and particularly, a method for inhibiting metastasis without causing any severe side effects, has been desired.

### Regarding TM7XN1 and GPR56

TM7XN1 and GPR56 are both human-derived GTP-binding protein coupled receptors (GPCRs: G-Protein Coupled Receptors). GPCRs are membrane proteins characterized by an amino acid sequence that contains 7 hydrophobic domains; that is, the 7-transmembrane region. GPCRs are seen in wide-ranging organisms. GPCRs respond to wide-ranging various substances including lipid analogues, amino acid derivatives, low molecular weight peptides, and other molecules and have various physiological activities (see e.g., Ruffolo and Hollinger (edited in 1995), G-Protein Coupled Transmembrane Signaling Mechanisms, CRC Press, Boca Raton, FL; Watson and Arkinstall (1994), The G-Protein Linked Receptor FactsBook, Academic Press, San Diego, CA; Peroutka (edited in 1994), G Protein-Coupled Receptors, CRC Press, Boca Raton, FL; and Houslay and Milligan (1990), G-Proteins as Mediators of Cellular Signaling Processes, Wiley & Sons, New York, NY; Dohlman et al., (1991), Ann. Rev. Biochem. 60: 653-688).

TM7XN1 cDNA was cloned from a cDNA library derived from human malignant melanoma in 1999 (see Zendman AJ et al., (1999), FEBS Lett. 446: 292-298). Moreover, at around the same time, GPR56 cDNA was cloned from a cDNA library derived from human heart by another group (see Liu M et al., (1999), Genomics 55: 296-305). TM7XN1 and GPR56 are structurally highly analogous to each other. The only 2 differences between TM7XN1 and GPR56 are: that (1) in the 1^{st} intracellular loop region from the N-terminal side, GPR56 has an inserted sequence comprising 6 amino acids that are absent in TM7XN1; and that (2) 3 amino acids located distant from each other exhibit polymorphism (2 out of the 3 locations are extracellular regions). Portions other than the aforementioned are completely identical to each other. Therefore, TM7XN1 and GPR56 are thought to be in a splicing variant relationship such that they are variants generated via transcription and translation from the same gene (hereinafter referred to as the GPR56 gene) on the genome. In addition, the inserted sequence comprising 6 amino acids that exist in GPR56 is located in an intracellular region. Hence, TM7XN1 and GPR56 are completely identical to each other in terms of extracellular region (excluding the portion exhibiting polymorphism). Accordingly, there is an extremely high possibility that an antibody recognizing TM7XN1 expressed on cell surfaces also recognizes GPR56 expressed on cell surfaces.

A common structural characteristic observed in both TM7XN1 and GPR56 is the extracellular region located on the extreme N-terminal side. The region comprises approximately as many as 400 amino acids and has a characteristic mucin-like protein structure to which many sugar chains can be added. It is thus suggested that TM7XN1 and GPR56 may be involved in intercellular adhesion. Furthermore, in TM7XN1 and GPR56, an amino acid sequence located around the 7-transmembrane region has homology to a secretin receptor and the extracellular region on the N-terminal side is long (approximately 400 or more amino acids). Hence, TM7XN1 and GPR56 are classified as members of LNB-TM7, a GPCR superfamily subgroup (Stacey M et al., (2000), Trends Biochem Sci. 25: 284-289).

Regarding TM7XN1 expression sites, Zendman et al. have revealed that TM7XN1 is expressed not only in normal human organs such as the brain, kidney, testis, prostate, and thyroid gland, but also in cell lines of various human cancer types such as malignant melanoma (A375, Bowes), colon cancer (Caco-2, HT29), carcinoma planoepitheliale (A431), and lymphoma (Molt-4). Furthermore, they have also reported an interesting result that metastasis ability of malignant melanoma inversely correlates with TM7XN1 expression level (see Zendman AJ et al., (1999), FEBS Lett. 446: 292-298). Furthermore, regarding GPR56 expression sites, Liu et al., have revealed that GPR56 is expressed in various normal organs such as the brain, heart, kidney, testis, pancreas, skeletal muscle, and thyroid gland (see Liu M et al., (1999), Genomics 55: 296-305). In addition, in the above reports, expression sites have been examined by the RT-PCR method or Northern blotting method. However, primers or probes used in such methods contain sequences common to TM7XN1 cDNA and GPR56 cDNA, and there is an extremely high possibility that they have reported the expression sites without distinguishing between TM7XN1 and GPR56.

Furthermore, the interaction of TM7XN1 (in the published report, exclusively referred to as GPR56) with a molecule referred to as Tetraspanin (e.g., CD9 or CD81) (see Little KD et al., (2004), Mol Biol Cell 15: 2375-2387) and the malformation of the human frontal cortex due to mutation of the GPR56 gene (see Piao X et al., (2004), Science 303: 2033-2036) have been successively reported recently. Only recently, the expression of TM7XN1 (in the published report, exclusively referred to as GPR56) in glioma cells and the suppression of cell adhesion to a culture plate by a recombinant protein within the extracellular region on the N-terminal side of TM7XN1 in a glioma cell culture system have also been reported (see Shashidhar S et al., (2005), Oncogene 24: 1673-1682). Moreover, it has also been shown that TM7XN1 (in the published report, exclusively referred to as GPR56) is a target gene that is induced by pVHL, an antioncogene product (see Maina EN et al., (2005), Oncogene 24: 4549-4558).

In addition, TM7XN1 has already been for the subject of a patent application with the title "Novel gene and protein down-regulated by metastatic human melanoma cells, production method, and use" (see JP Patent Publication (Kokai) No. 2000-217585 A).

Furthermore, cDNA of Cyt28 which is a mouse homolog of TM7XN1 or GPR56 has been cloned as one of genes regulating hematopoietic stem cells (Phillips RL et al., (1999), GenBank Accession Number AF166382) and has been the subject of a patent application (see International Publication No. WO 00/11168 pamphlet). Furthermore, another group has identified Cyt28 cDNA as being among the genes that are expressed at higher levels in mouse bone marrow hematopoietic stem cells than those in the whole cells of the mouse bone marrow (through comparison of the expression levels of the two) (see Terskikh AJ et al., (2001), Proc. Natl. Acad. Sci. U.S.A. 98: 7934-7939). Cyt28 is GPCR having approximately 75% homology (amino acid sequence) with TM7XN1 or GPR56. Cyt28 has molecular characteristics similar to those of TM7XN1 or GPR56 in terms of the amino acid sequence located around the extracellular region located on the N-terminal side or the 7-transmembrane region, for example. Thus, Cyt28 is classified as LNB-TM7. Cyt28 expression has also been confirmed in mouse fetal liver hematopoietic stem cells. Furthermore, the likely expression of Cyt28 in mouse neural stem cells has also been confirmed (see Terskikh AJ et al., (2001), Proc. Natl. Acad. Sci. U.S.A. 98: 7934-7939).

### Disclosure of the Invention

### Objects to be Achieved by the Invention

Objects of the present invention are to provide a novel method for inhibiting metastasis of various cancer cells expressing TM7XN1, a specified G-protein coupled receptor, using an antibody against TM7XN1 or an antibody acting as an antagonist, for example, and to provide a pharmaceutical composition to be used for such method.

### Means to Achieve the Objects

As described above, although various findings concerning TM7XN1 are increasingly accumulated, a physiological ligand of TM7XN1 has remained unidentified. Hence, the physiological roles of TM7XN1 have not yet been completely elucidated.

The present inventors have prepared a monoclonal antibody KSA7 that binds to TM7XN1 for the purpose of elucidating the physiological functions of TM7XN1. The present inventors then examined the effects of the antibody against various pathological models. As one of such efforts, the present inventors have also examined the effects of the antibody using a bone metastasis model (established by transplanting a TM7XN1-expressing human acute myeloid leukemia cell line (KG1a) into the veins of immunodeficient mice to cause bone marrow metastasis) based on Zendman et al's finding such that metastasis ability of malignant melanoma and TM7XN1 expression level inversely correlate with each other. Thus, a result unpredictable from Zendman et al's findings has been obtained, such that compared with a control group, metastasis to the bone marrow has been significantly suppressed in a KSA7-administered group.

The present inventors further additionally prepared monoclonal antibodies 4M2B, 7M1, and 21M1, which bind to TM7XN1. The present inventors have also examined the effects of 4M2B, 7M1, and 21M1 simultaneously with KSA7 using another bone metastasis model (established by transplanting a TM7XN1-expressing human acute myeloid leukemia cell line (KG1a) into the veins of immunodeficient mice to cause bone marrow metastasis). Thus, results were obtained to the effect that metastasis to the bone marrow had been significantly suppressed in groups to which 3 clones other than KSA7 had been administered. Therefore, it has been demonstrated that not only a specific monoclonal antibody but also monoclonal antibodies that bind to TM7XN1 can possess such activity. It has also been newly demonstrated that a TM7XN1 molecule is an important target molecule for suppression of metastasis to the bone marrow.

Moreover, the present inventors have further conducted examinations as follows using 21M1 exerting the highest activity among the KSA7, 4M2B, 7M1, and 21M1 clones in the above bone metastasis model.

First, the present inventors examined the effects of 21M 1 using a bone metastasis model (established by transplanting a TM7XN1-expressing human multiple myeloma cell line (RPMI8226) into the veins of immunodeficient mice to cause bone marrow metastasis). As a result, the tendency of 21M1 to suppress metastases to the bone marrow has been demonstrated.

Furthermore, the present inventors examined the effects of 21M1 using an orthotopic transplantation · metastasis model established by transplanting a tumor mass of a TM7XN1-expressing and GFP-labeled human pancreatic cancer cell line (BxPC3) into the pancreas (orthotopic) of immunodeficient mice, allowing the survival of the primary lesion, and thus causing systemic metastasis. As a result, the tendency of 21M1 to suppress metastasis of pancreatic cancer cells to the spleen has been demonstrated.

As described above, it has been demonstrated that monoclonal antibodies binding to TM7XN1 may suppress metastasis of various tumors including acute myeloid leukemia, multiple myeloma, and pancreatic cancer. It has newly been demonstrated that the TM7XN1 molecule is an important target molecule for suppressing metastasis of various tumors.

Since TM7XN1 has a mucin-like protein structure, its possible involvement in intercellular adhesion has been suggested. However, findings concerning its involvement in cancer cell metastasis or the like have not been specifically confirmed to date. It has been demonstrated for the first time by the examples in the present invention that TM7XN1 may be involved in metastasis of tumor cells and that antibodies against TM7XN1 may be useful for suppression of metastasis of cancer cells expressing TM7XN1. Furthermore, the present inventors have also discovered that such antibodies exert a clear effect of suppressing metastasis without causing any severe side effects. Thus, the present inventors have completed the present invention.

Embodiments of the present invention are as follows.
[1] A cancer-metastasis-suppressing agent, comprising an antibody against TM7XN1 as an active ingredient.
[2] The cancer-metastasis-suppressing agent according to [1], wherein a cell of the cancer expresses TM7XN1 on its surface.
[3] The cancer-metastasis-suppressing agent according to [1] or [2], wherein the cancer can metastasize to the bone.
[4] The cancer-metastasis-suppressing agent according to [1] or [2], wherein the cancer can metastasize to the spleen.
[5] The cancer-metastasis-suppressing agent according to any one of [1] to [4], wherein the cancer is selected from the group consisting of mammary cancer, prostate cancer, lung cancer, thyroid gland cancer, gastric cancer, malignant melanoma, neuroblastoma, multiple myeloma, acute myeloid leukemia, and pancreatic cancer.
[6] The cancer-metastasis-suppressing agent according to any one of [1] to [5], being capable of suppressing the metastasis of a cancer cell to the bone marrow.
[7] The cancer-metastasis-suppressing agent according to any one of [1] to [5], being capable of suppressing the metastasis of a cancer cell to the spleen.
[8] A method for suppressing a cancer metastasis, comprising administering the cancer-metastasis-suppressing agent according to any one of [1] to [7] to a non-human animal with cancer.

This description includes part or all of the contents as disclosed in the description and/or drawings of Japanese Patent Application No. 2004-330814, which is a priority document of the present application.

### Effect of the Invention

According to the present invention, treatment with an antibody against TM7XN1 is performed against various cancer cells expressing TM7XN1, the specified G-protein coupled cell surface receptor, so that metastasis of cancer cells expressing TM7XN1, and particularly, metastasis to other organs such as the bone marrow and the spleen, are suppressed. It is expected that such suppression will lead to quality improvement in cancer treatment and improvement in patient QOL.

### Brief Description of the Drawings

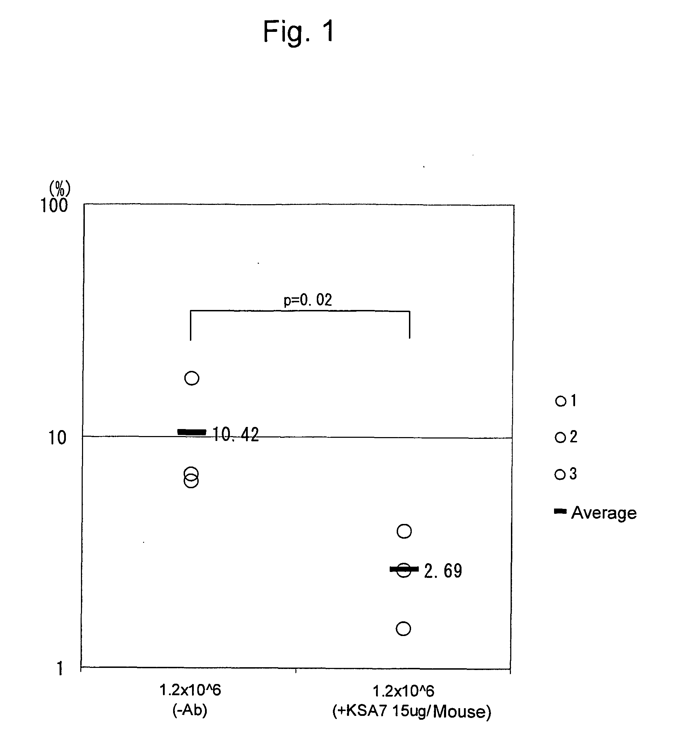

Fig. 1 shows the metastasis-suppressing effect of an anti-TM7XN1 monoclonal antibody KSA7 in a bone metastasis model, which is represented by % of chimerism in the bone marrow.

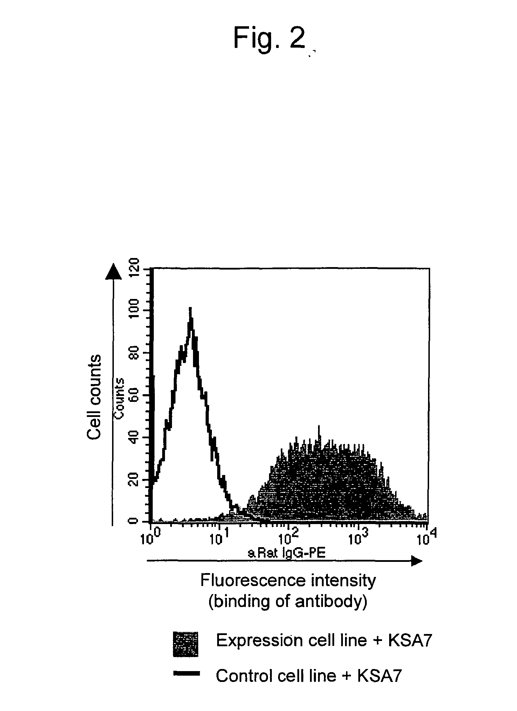

Fig. 2 shows the activity of an anti-TM7XN1 monoclonal antibody KSA7 to bind to TM7XN1, which is shown via comparison of TM7XN1-expressing cells with cells not expressing TM7XN1.

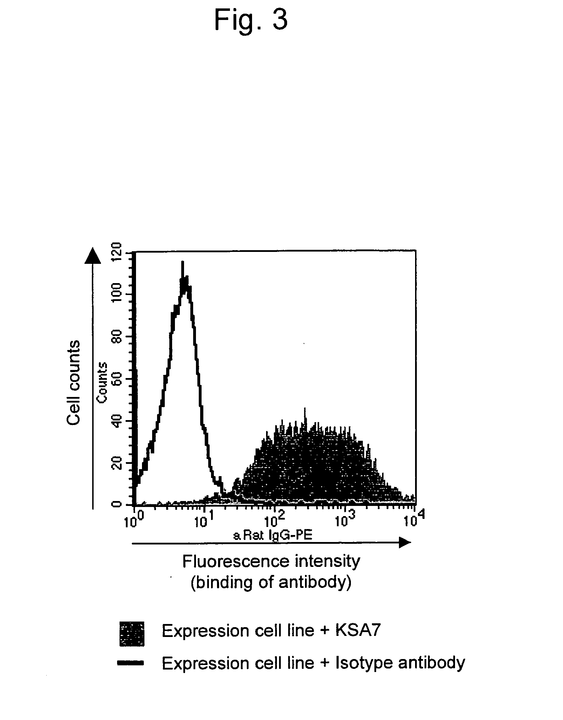

Fig. 3 shows the activity of the anti-TM7XN1 monoclonal antibody KSA7 to bind to TM7XN1, which is shown via comparison of KSA7 with an isotype antibody.

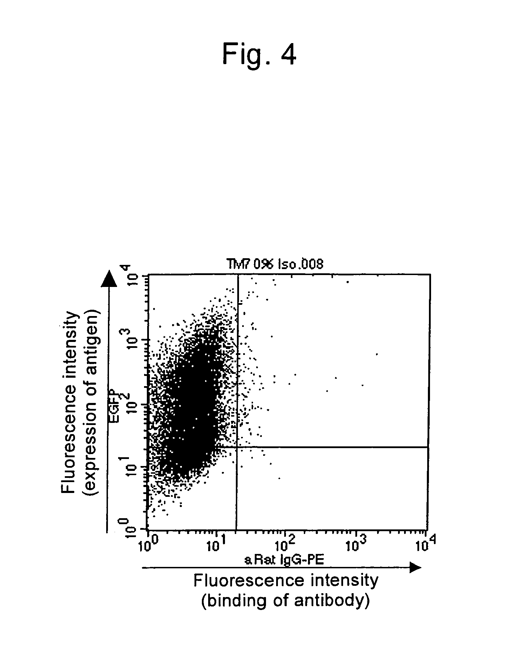

Fig. 4 shows correlation between the expression levels of a TM7XN1-EGFP fusion-protein and the binding amounts of the isotype antibody.

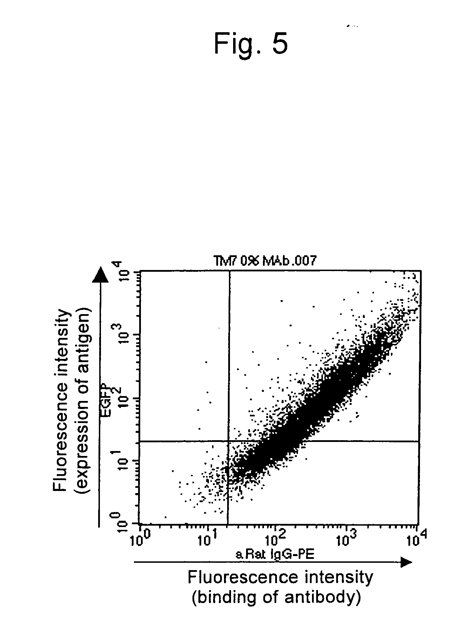

Fig. 5 shows correlation between the expression levels of a TM7XN1-EGFP fusion-protein and the binding amounts of KSA7.

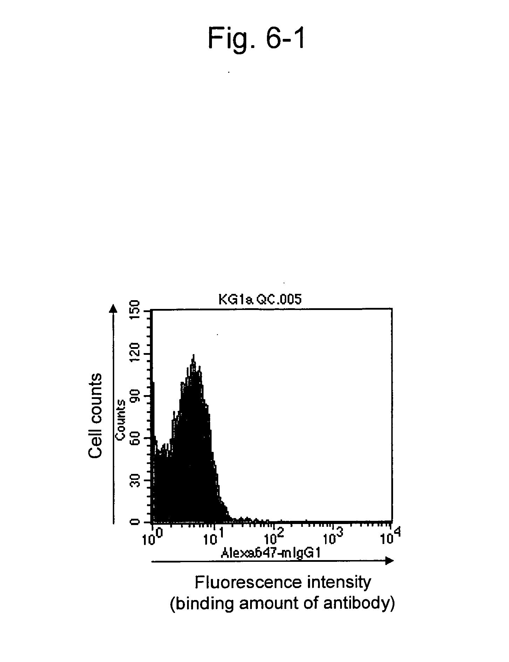

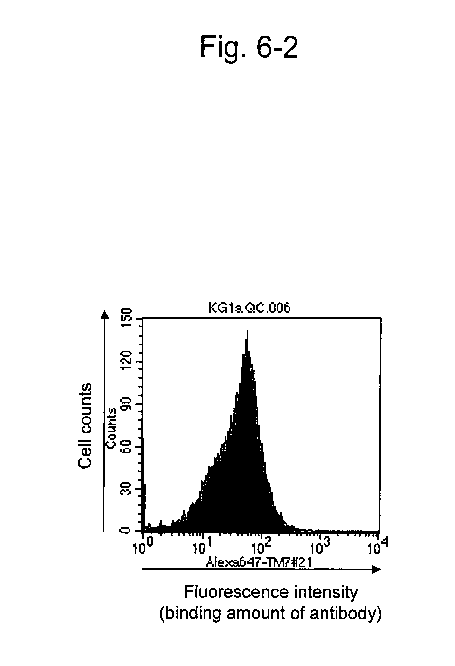

Fig. 6-1 shows TM7XN1 expression in KG1a cells, which is shown via comparison of the binding activity of isotype control antibodies with that of an anti-TM7XN1 monoclonal antibody 21M1. The amounts of the isotype control antibodies binding to the KG1a cells are represented by fluorescence intensities.
Fig. 6-2 shows TM7XN1 expression in KG1a cells, which is shown via comparison of the binding activity of isotype control antibodies with that of an anti-TM7XN 1 monoclonal antibody 21M1. The amounts of 21MI binding to the KG1a cells are represented by fluorescence intensities.

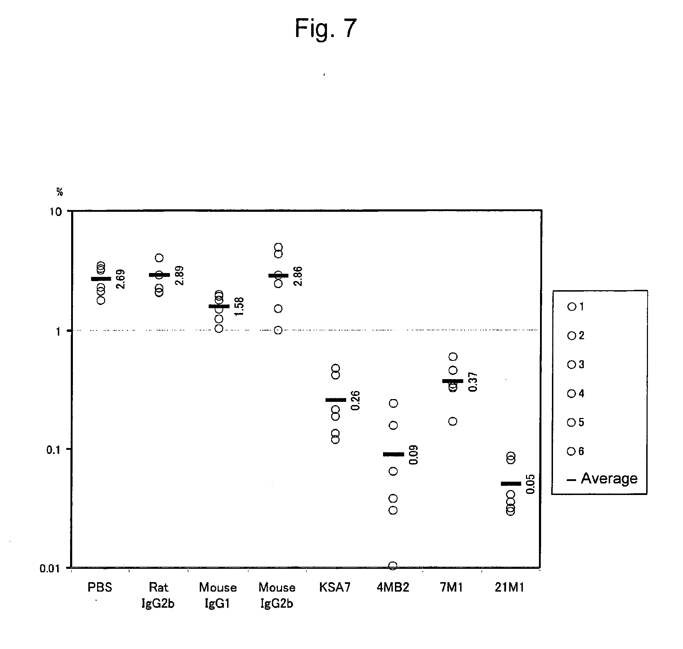

Fig. 7 shows the metastasis-suppressing effect of each of a plurality of clones (KSA7, 4M2B, 7M1, and 21M1) of the anti-TM7XN1 monoclonal antibody in a bone metastasis model established using KG1a cells, which is represented by % of chimerism in the bone marrow.

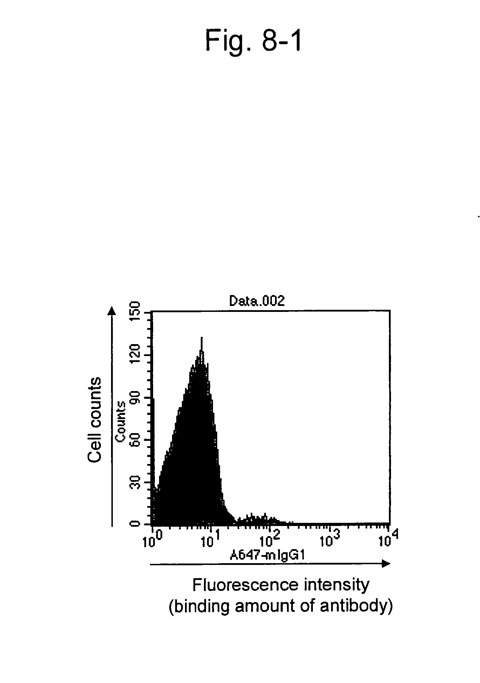

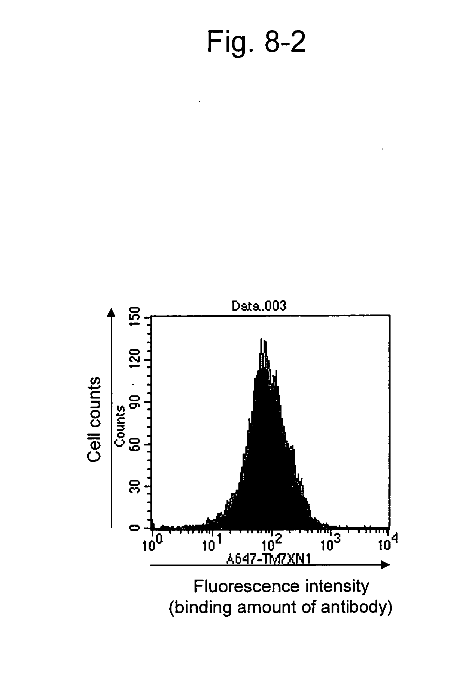

Fig. 8-1 shows TM7XN1 expression in RPMI8226 cells, which is shown via comparison of the binding activity of an isotype control antibody with that of an anti-TM7XN1 monoclonal antibody 21M1. The amounts of the isotype control antibody binding to the RPMI8226 cells are represented by fluorescence intensities.
Fig. 8-2 shows TM7XN1 expression in RPMI8226 cells, which is shown via comparison of the binding activity of an isotype control antibody with that of an ariti-TM7XN1 monoclonal antibody 21M1. The amounts of 21M1 binding to the RPMI8226 cells are represented by fluorescence intensities.

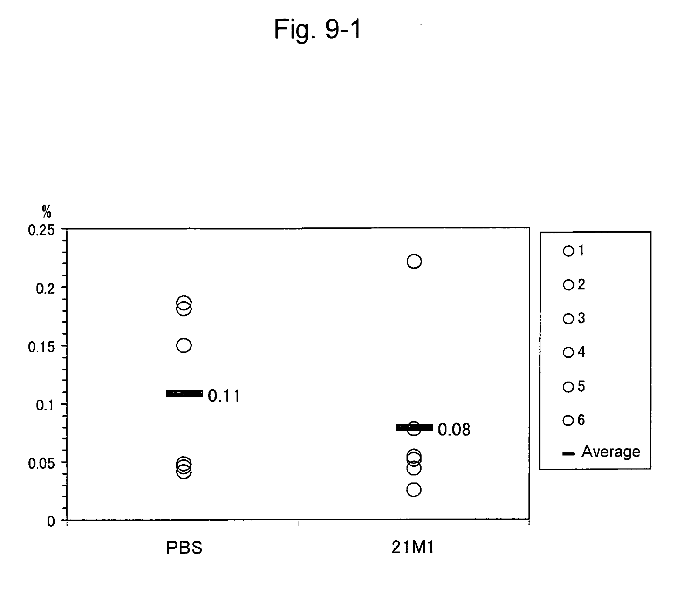

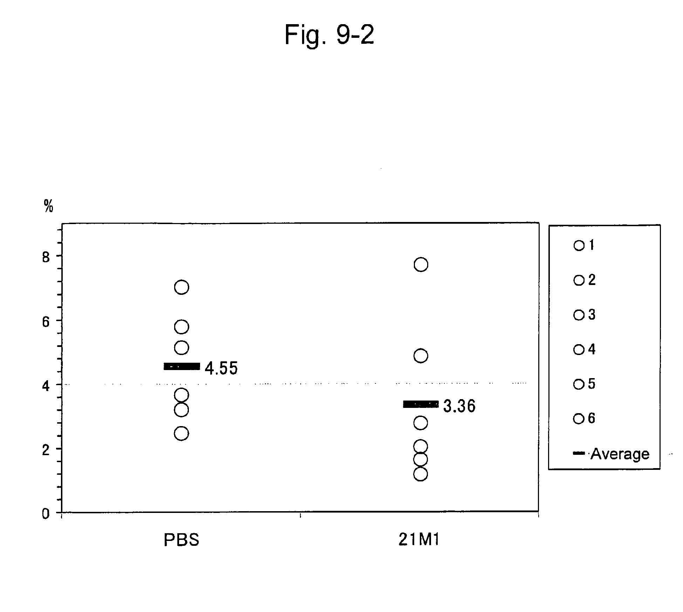

Fig. 9-1 shows the effect of an anti-TM7XN1 monoclonal antibody 21M1 in a metastasis model established using RPMI8226. The effect of the anti-TM7XN1 monoclonal antibody 21M1 to suppress bone metastasis is represented by % of chimerism in the bone marrow.
Fig. 9-2 shows the effect of an anti-TM7XN1 monoclonal antibody 21M1 in a metastasis model established using RPMI8226. The effect of the anti-TM7XN1 monoclonal antibody 21M1 to suppress spleen metastasis is represented by % of chimerism in the spleen.

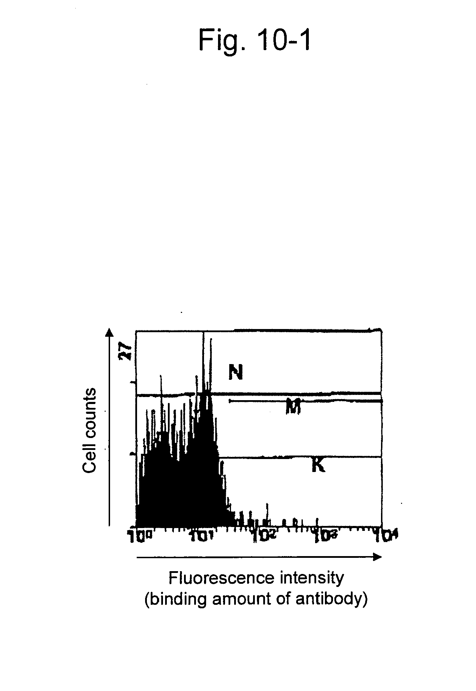

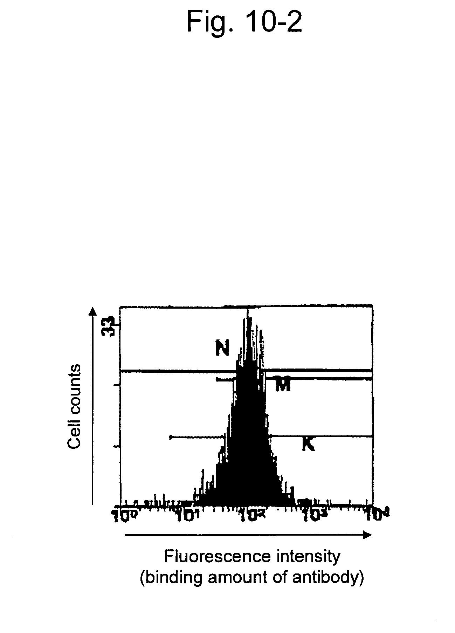

Fig. 10-1 shows TM7XN1 expression in a pancreatic cancer cell line BxPC-3, which is shown via comparison of the binding activity of an isotype control antibody with that of an anti-TM7XN1 monoclonal antibody 21M1. The amounts of the isotype control antibody binding to the pancreatic cancer cell line BxPC-3 are represented by fluorescence intensities.
Fig. 10-2 shows TM7XN1 expression in a pancreatic cancer cell line BxPC-3, which is shown via comparison of the binding activity of an isotype control antibody with that of an anti-TM7XN1 monoclonal antibody 21M1. The amounts of 21M 1 binding to the pancreatic cancer cell line BxPC-3 are represented by fluorescence intensities.

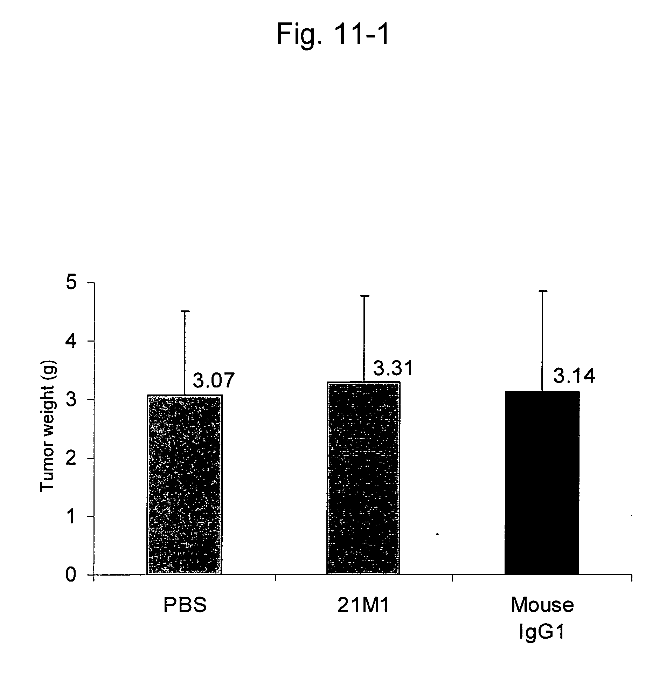

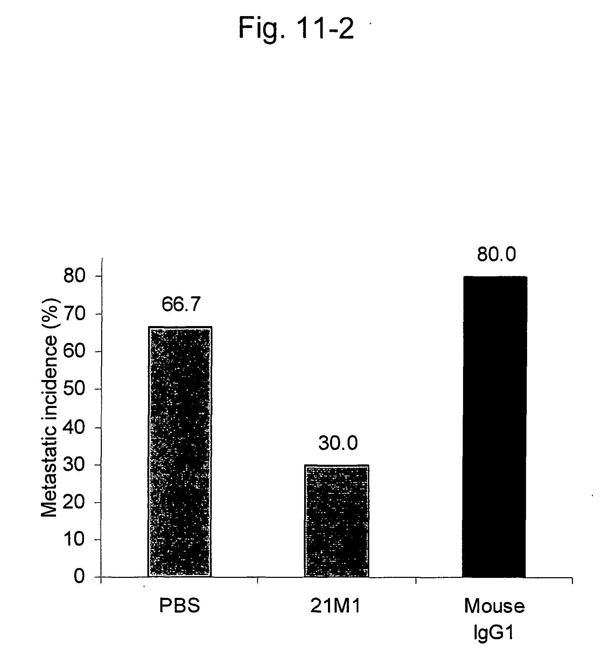

Fig. 11-1 shows the effect of an anti-TM7XN1 monoclonal antibody 21M1 in a model for visually detecting metastasis that has taken place after the orthotopic transplantation of solid cancer cells. The effect of the anti-TM7XN1 monoclonal antibody 21M1 on the involution of a primary lesion is represented by the weight of such primary lesion.
Fig. 11-2 shows the effect of an anti-TM7XN1 monoclonal antibody 21M1 in a model for visually detecting metastasis that has taken place after orthotopic transplantation of solid cancer cells. The effect of the anti-TM7XN1 monoclonal antibody 21M1 on spleen metastasis is represented by the incidence % of spleen metastasis.

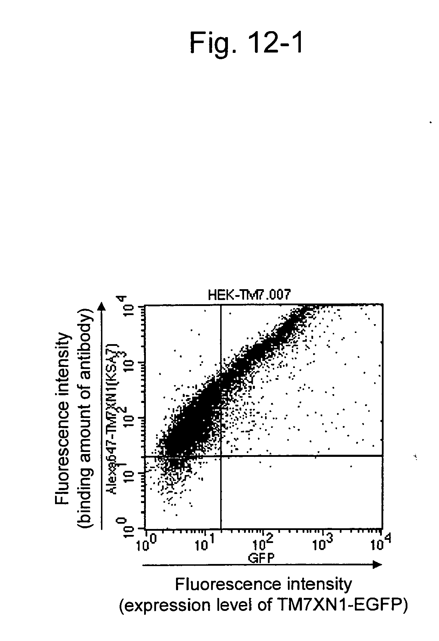

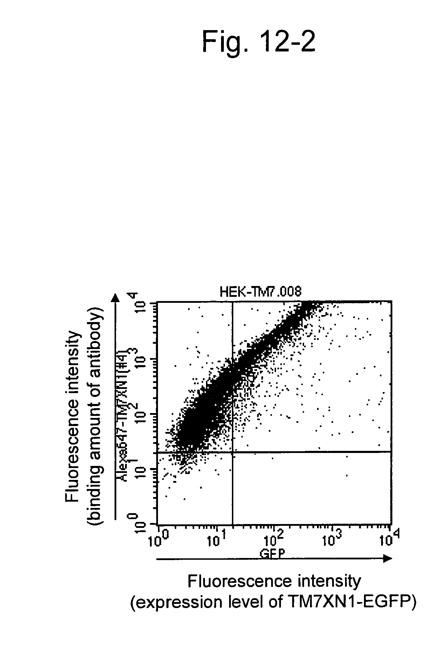

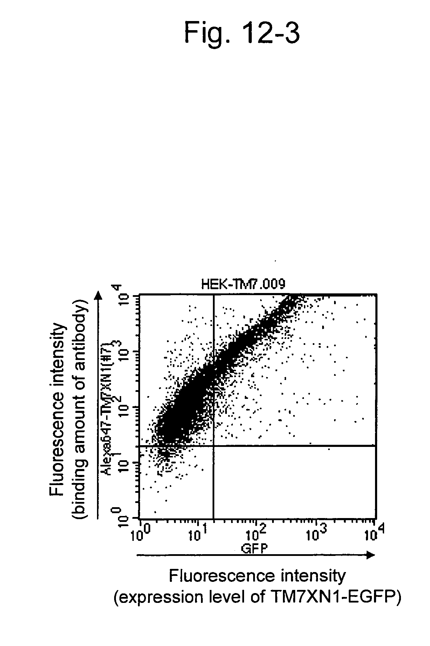

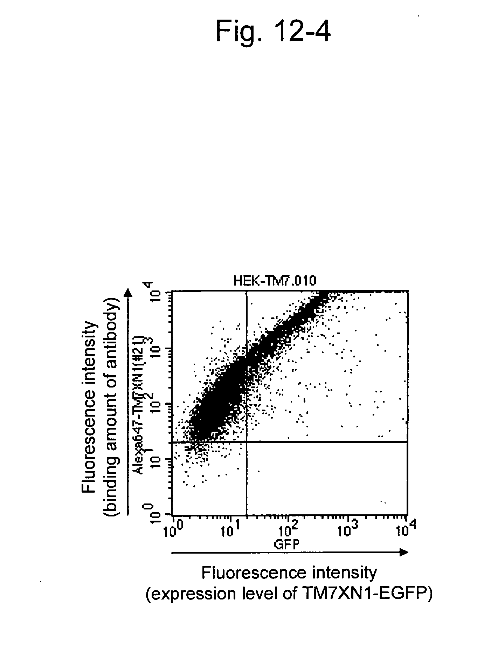

Fig. 12-1 shows correlation between the expression levels of a TM7XN1-EGFP fusion protein and the binding amounts of an anti-TM7XN1 monoclonal antibody. Fig. 12-1 also shows correlation between the expression levels of the TM7XN1-EGFP fusion protein and the binding amounts of KSA7.
Fig. 12-2 shows correlation between the expression levels of a TM7XN1-EGFP fusion protein with the binding amounts of an anti-TM7XN 1 monoclonal antibody. Fig. 12-2 also shows correlation between the expression levels of the TM7XN1-EGFP fusion protein and the binding amounts of 4M2B.
Fig. 12-3 shows correlation between the expression levels of a TM7XN1-EGFP fusion protein and the binding amounts of an anti-TM7XN 1 monoclonal antibody. Fig. 12-3 also shows correlation between the expression levels of the TM7XN1-EGFP fusion protein and the binding amounts of 7M1.
Fig. 12-4 shows correlation between the expression levels of a TM7XN1-EGFP fusion protein and the binding amounts of an anti-TM7XN1 monoclonal antibody. Fig. 12-4 also shows correlation between the expression levels of the TM7XN1-EGFP fusion protein and the binding amounts of 21 M 1.

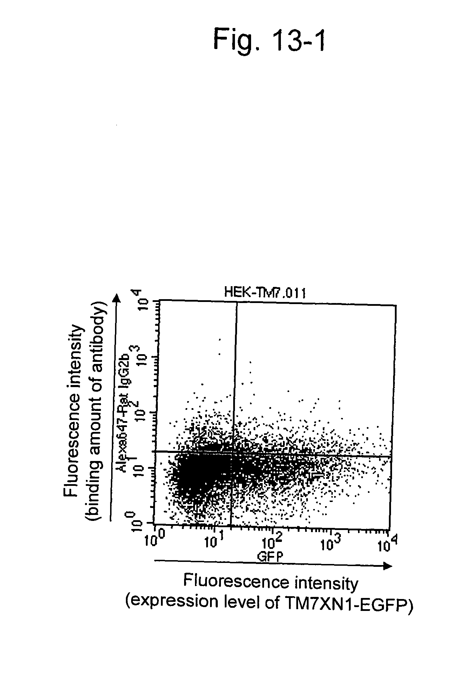

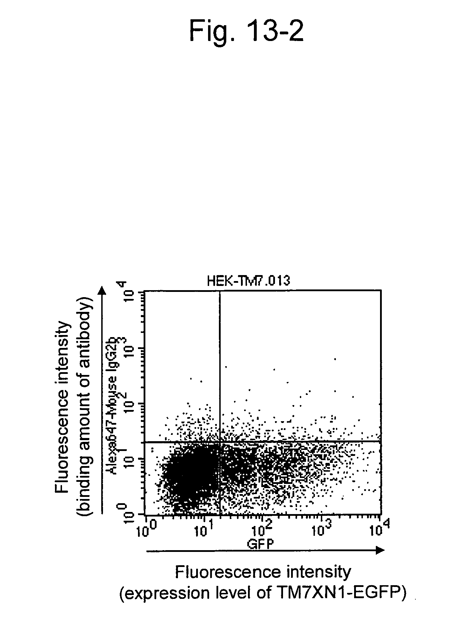

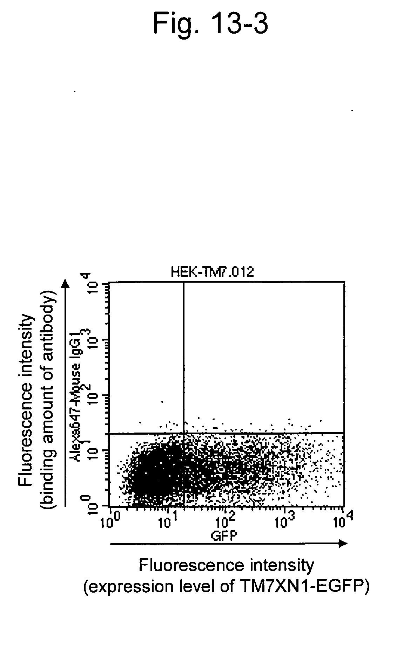

Fig. 13-1 shows correlation between the expression levels of a TM7XN1-EGFP fusion protein and the binding amounts of isotype control antibodies. Fig. 13-1 also shows correlation between the expression levels of the TM7XN1-EGFP fusion protein and the binding amounts of rat IgG2b.
Fig. 13-2 shows correlation between the expression levels of a TM7XN1-EGFP fusion protein and the binding amounts of isotype control antibodies. Fig. 13-2 also shows correlation between the expression levels of a TM7XN1-EGFP fusion protein and the binding amounts of mouse IgG2b.
Fig. 13-3 shows correlation between the expression levels of a TM7XN1-EGFP fusion protein and the binding amounts of isotype control antibodies. Fig. 13-3 also shows correlation between the expression levels of the TM7XN1-EGFP fusion protein and the binding amounts of mouse IgG1.

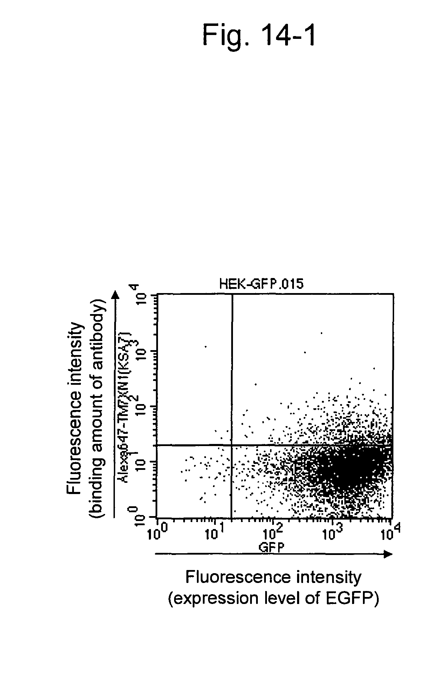

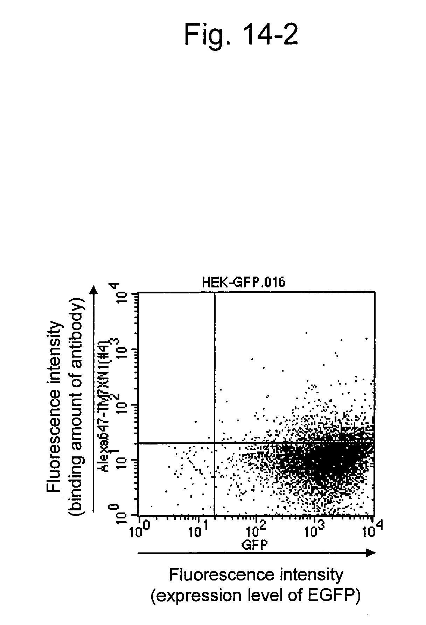

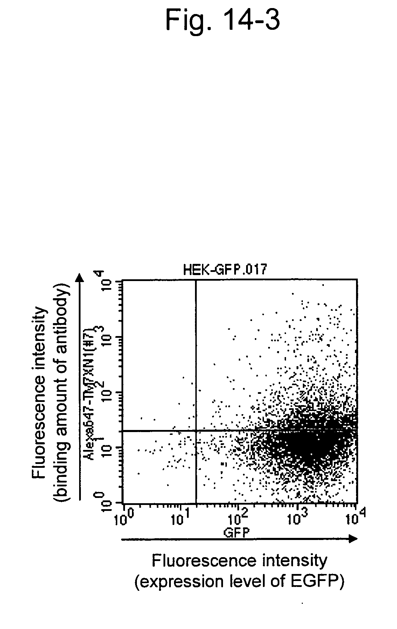

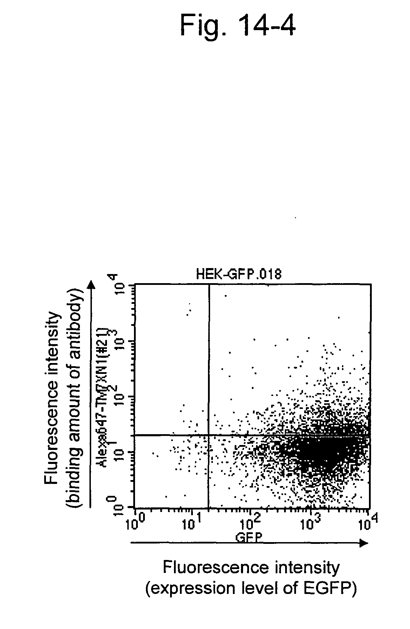

Fig. 14-1 shows correlation between the expression levels of EGFP and the binding amounts of an anti-TM7XN1 monoclonal antibody. Fig. 14-1 also shows correlation between the expression levels of EGFP and the binding amounts of KSA7.
Fig. 14-2 shows correlation between the expression levels of EGFP and the binding amounts of an anti-TM7XN1 monoclonal antibody. Fig. 14-2 also shows correlation between the expression levels of EGFP and the binding amounts of 4M2B.
Fig. 14-3 shows correlation between the expression levels of EGFP and the binding amounts of an anti-TM7XN1 monoclonal antibody. Fig. 14-3 also shows correlation between the expression levels of EGFP and the binding amounts of 7M1.
Fig. 14-4 shows correlation between the expression levels of EGFP and the binding amounts of an anti-TM7XN1 monoclonal antibody. Fig. 14-4 also shows correlation between the expression levels of EGFP and the binding amounts of 21M1.

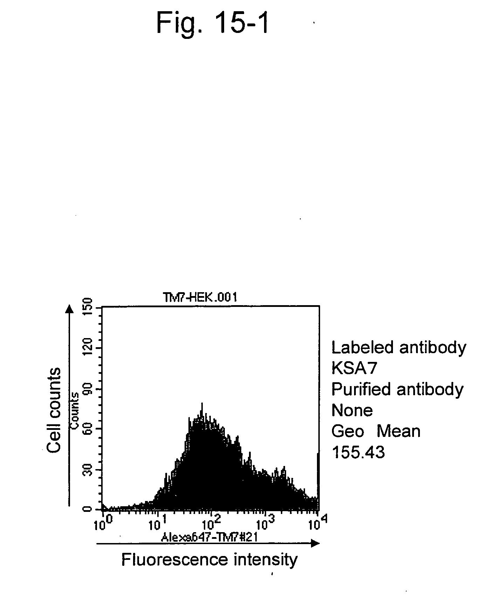

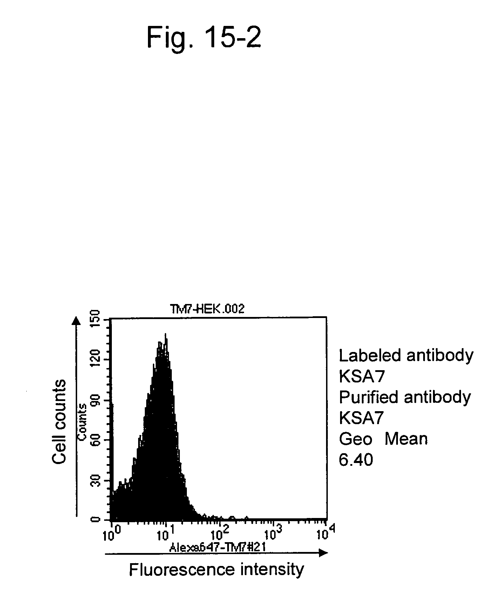

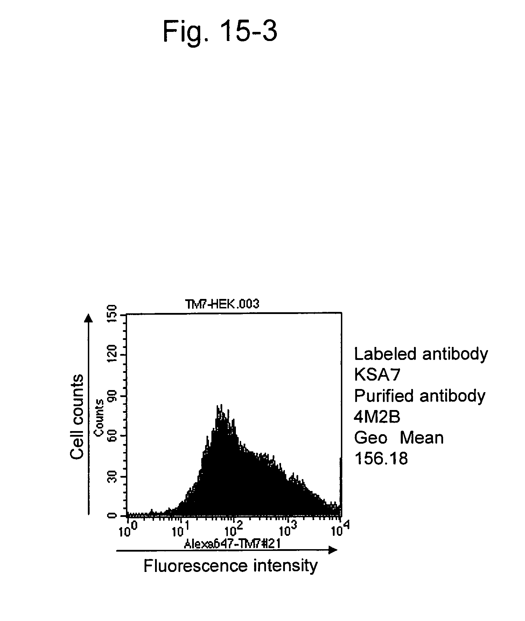

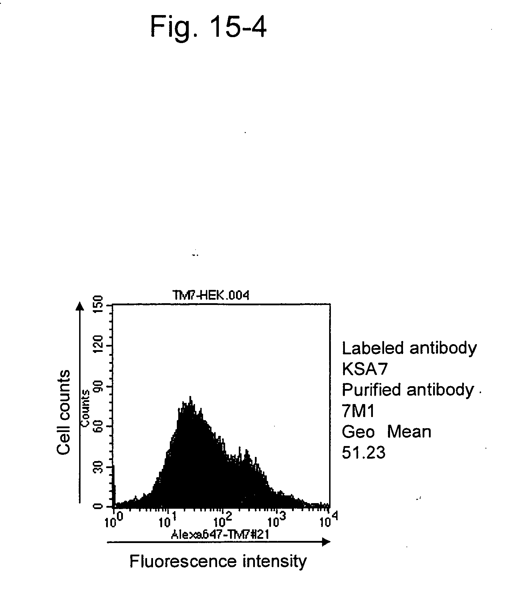

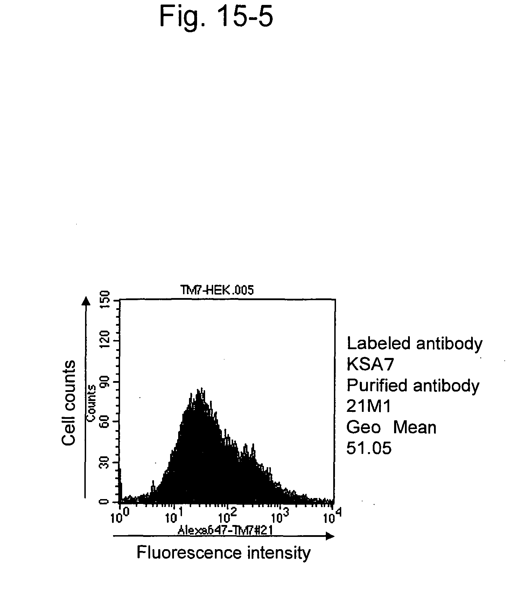

Fig. 15-1 shows competitiveness of KSA7 (labeled antibody), which is an anti-TM7XN1 monoclonal antibody, with KSA7, 4M2B, 7M1, or 21M1 (purified antibody). Fig. 15-1 also shows the binding amounts (fluorescence intensities) of KSA7 (labeled antibody) when a TM7XN1-EGFP fusion-protein-expressing cell line has been stained with KSA7 (labeled antibody) alone.
Fig. 15-2 shows competitiveness of KSA7 (labeled antibody), which is an anti-TM7XN1 monoclonal antibody, with KSA7, 4M2B, 7M1, or 21M1 (purified antibody). Fig. 15-2 also shows the binding amounts (fluorescence intensities) of KSA7 (labeled antibody) when a TM7XN1-EGFP fusion-protein-expressing cell line has been stained with KSA7 (labeled antibody) and KSA7 (purified antibody).
Fig. 15-3 shows competitiveness of KSA7 (labeled antibody), which is an anti-TM7XN1 monoclonal antibody, with KSA7, 4M2B, 7M1, or 21M1 (purified antibody). Fig. 15-3 also shows the binding amounts (fluorescence intensities) of KSA7 (labeled antibody) when a TM7XN1-EGFP fusion-protein-expressing cell line has been stained with KSA7 (labeled antibody) and 4M2B (purified antibody).
Fig. 15-4 shows competitiveness of KSA7 (labeled antibody), which is an anti-TM7XN1 monoclonal antibody, with KSA7, 4M2B, 7M1, or 21M1 (purified antibody). Fig. 15-4 also shows the binding amounts (fluorescence intensities) of KSA7 (labeled antibody) when a TM7XN1-EGFP fusion-protein-expressing cell line has been stained with KSA7 (labeled antibody) and 7M1 (purified antibody).
Fig. 15-5 shows competitiveness of KSA7 (labeled antibody), which is an anti-TM7XN1 monoclonal antibody, with KSA7, 4M2B, 7M1, or 21M1 (purified antibody). Fig. 15-5 also shows the binding amounts (fluorescence intensities) of KSA7 (labeled antibody) when a TM7XN1-EGFP fusion-protein-expressing cell line has been stained with KSA7 (labeled antibody) and 21M1 (purified antibody).

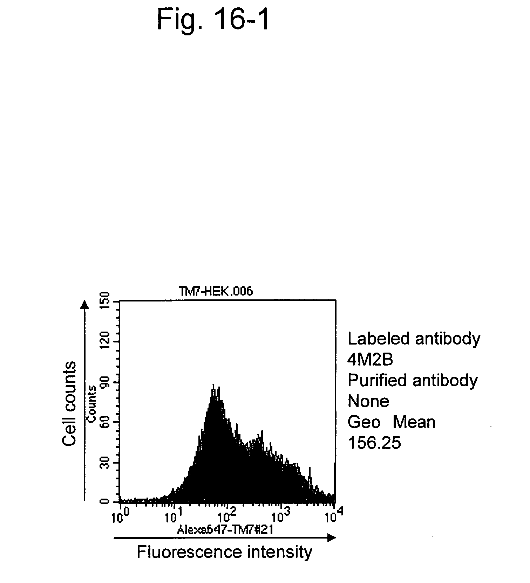

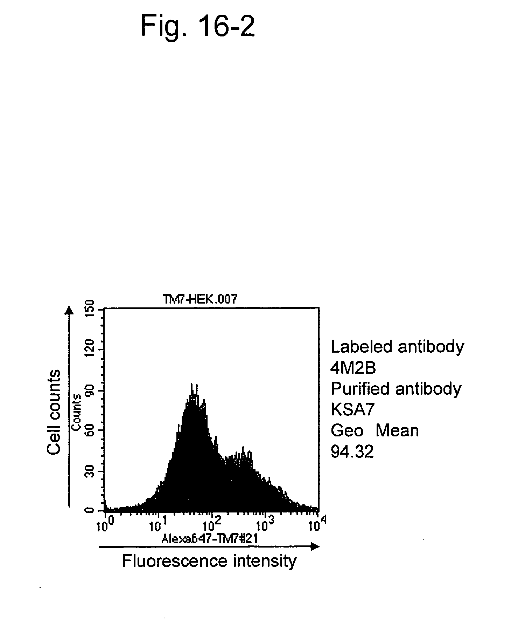

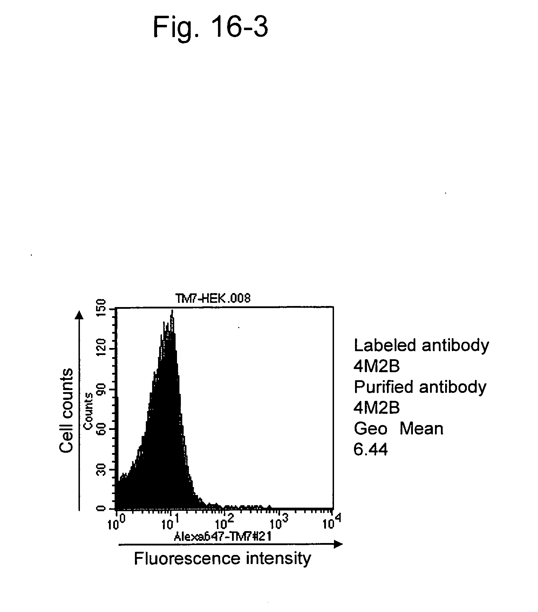

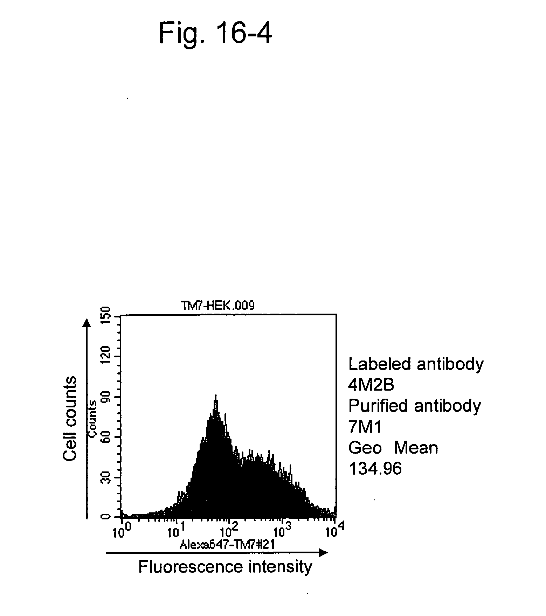

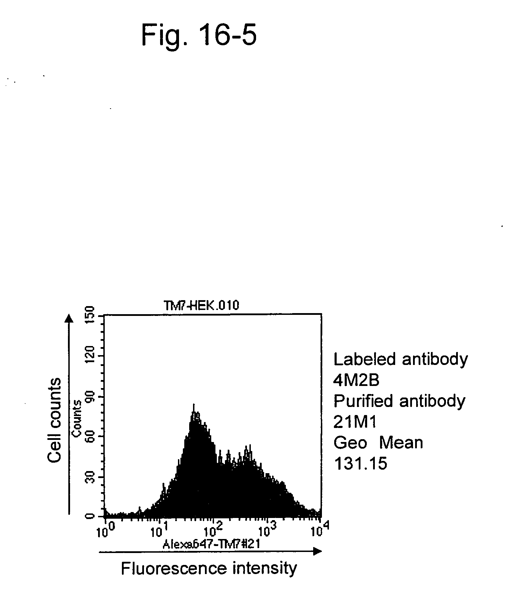

Fig. 16-1 shows competitiveness of 4M2B (labeled antibody), which is an anti-TM7XN monoclonal antibody, with KSA7, 4M2B, 7M 1, or 21M1 (purified antibody). Fig. 16-1 also shows the binding amounts (fluorescence intensities) of 4M2B (labeled antibody) when a TM7XN1-EGFP fusion-protein-expressing cell line has been stained with 4M2B (labeled antibody) alone.
Fig. 16-2 shows competitiveness of 4M2B (labeled antibody), which is an anti-TM7XN1 monoclonal antibody, with KSA7, 4M2B, 7M1, or 21M1 (purified antibody). Fig. 16-2 also shows the binding amounts (fluorescence intensities) of 4M2B (labeled antibody) when a TM7XN1-EGFP fusion-protein-expressing cell line has been stained with 4M2B (labeled antibody) and KSA7 (purified antibody).
Fig. 16-3 shows competitiveness of 4M2B (labeled antibody), which is an anti-TM7XN1 monoclonal antibody, with KSA7, 4M2B, 7M1, or 21M1 (purified antibody). Fig. 16-3 also shows the binding amounts (fluorescence intensities) of 4M2B (labeled antibody) when a TM7XN1-EGFP fusion-protein-expressing cell line has been stained with 4M2B (labeled antibody) and 4M2B (purified antibody).
Fig. 16-4 shows competitiveness of 4M2B (labeled antibody), which is an anti-TM7XN1 monoclonal antibody, with KSA7, 4M2B, 7M 1, or 21M1 (purified antibody). Fig. 16-4 shows the binding amounts (fluorescence intensities) of 4M2B (labeled antibody) when a TM7XN1-EGFP fusion-protein-expressing cell line has been stained with 4M2B (labeled antibody) and 7M1 (purified antibody).
Fig. 16-5 shows competitiveness of 4M2B (labeled antibody), which is an anti-TM7XN 1 monoclonal antibody, with KSA7, 4M2B, 7M 1, or 21M1 (purified antibody). Fig. 16-5 also shows the binding amounts (fluorescence intensities) of 4M2B (labeled antibody) when a TM7XN1-EGFP fusion-protein-expressing cell line has been stained with 4M2B (labeled antibody) and 21M1 (purified antibody).

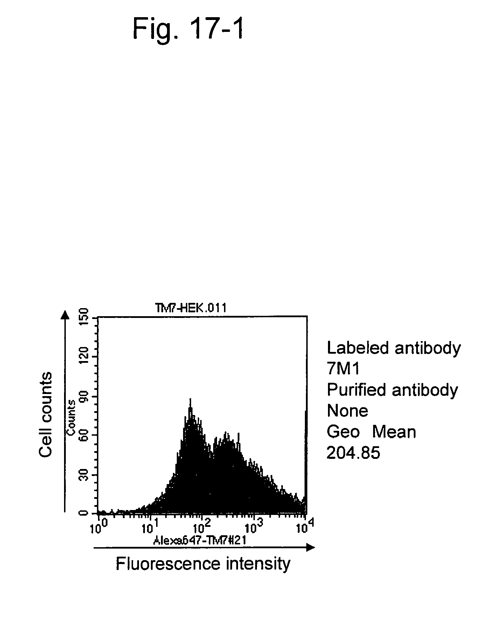

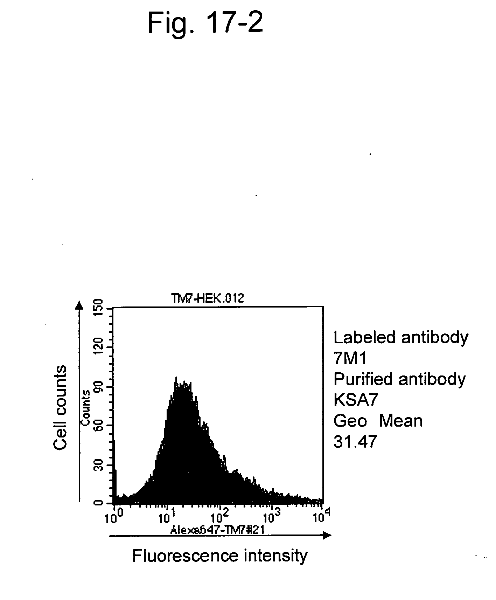

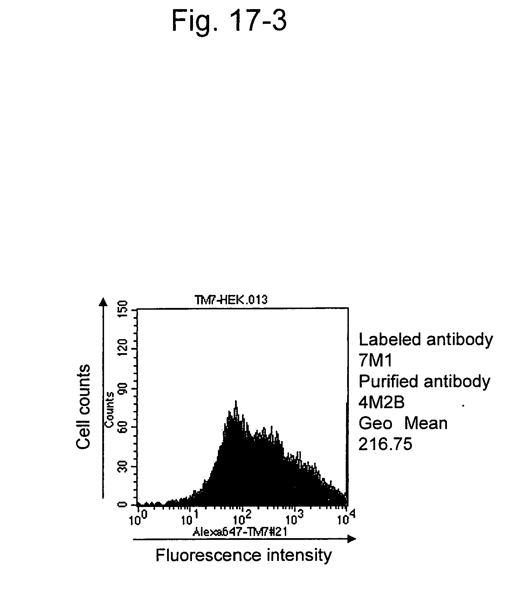

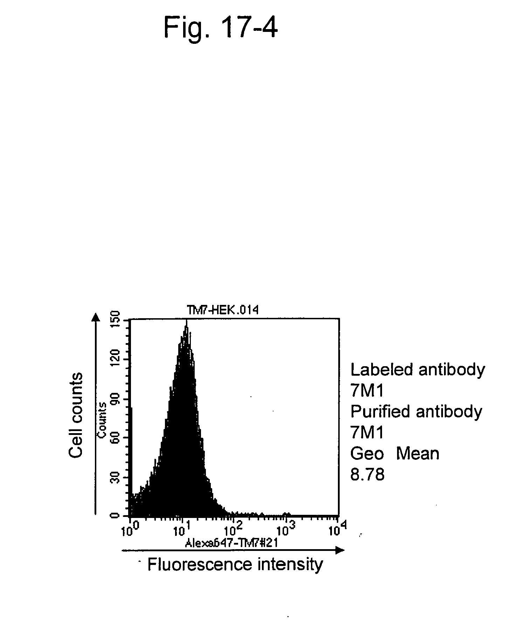

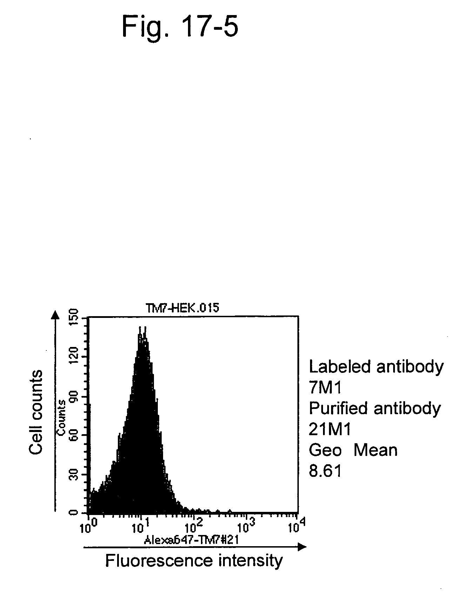

Fig. 17-1 shows competitiveness of 7M1 (labeled antibody), which is an anti-TM7XN 1 monoclonal antibody, with KSA7, 4M2B, 7M1, or 21M 1 (purified antibody). Fig. 17-1 also shows the binding amounts (fluorescence intensities) of 7M1 (labeled antibody) when a TM7XN1-EGFP fusion-protein-expressing cell line has been stained with 7M1 (labeled antibody) alone.
Fig. 17-2 shows competitiveness of 7M1 (labeled antibody), which is an anti-TM7XN1 monoclonal antibody, with KSA7, 4M2B, 7M1, or 21M1 (purified antibody). Fig. 17-2 also shows the binding amounts (fluorescence intensities) of 7M1 (labeled antibody) when a TM7XN1-EGFP fusion-protein-expressing cell line has been stained with 7M1 (labeled antibody) and KSA7 (purified antibody).
Fig. 17-3 shows competitiveness of 7M1 (labeled antibody), which is an anti-TM7XN1 monoclonal antibody, with KSA7, 4M2B, 7M1, or 21M1 (purified antibody). Fig. 17-3 also shows the binding amounts (fluorescence intensities) of 7M1 (labeled antibody) when a TM7XN1-EGFP fusion-protein-expressing cell line has been stained with 7M1 (labeled antibody) and 4M2B (purified antibody).
Fig. 17-4 shows competitiveness of 7M1 (labeled antibody), which is an anti-TM7XN1 monoclonal antibody, with KSA7, 4M2B, 7M1, or 21M1 (purified antibody). Fig. 17-4 also shows the binding amounts (fluorescence intensities) of 7M1 (labeled antibody) when a TM7XN1-EGFP fusion-protein-expressing cell line has been stained with 7M1 (labeled antibody) and 7M1 (purified antibody).
Fig. 17-5 shows competitiveness of 7M1 (labeled antibody), which is an anti-TM7XN1 monoclonal antibody, with KSA7, 4M2B, 7M1, or 21M1 (purified antibody). Fig. 17-5 also shows the binding amounts (fluorescence intensities) of 7M1 (labeled antibody) when a TM7XN1-EGFP fusion-protein-expressing cell line has been stained with 7M1 (labeled antibody) and 21M1 (purified antibody).

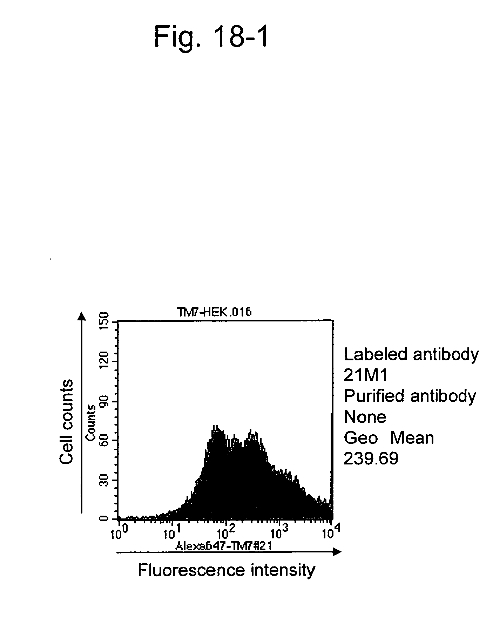

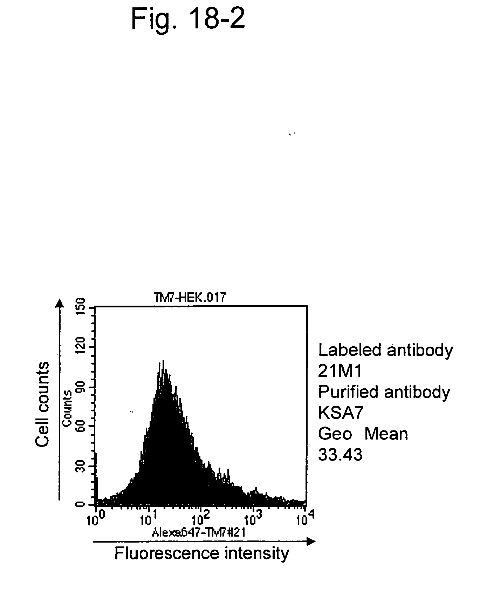

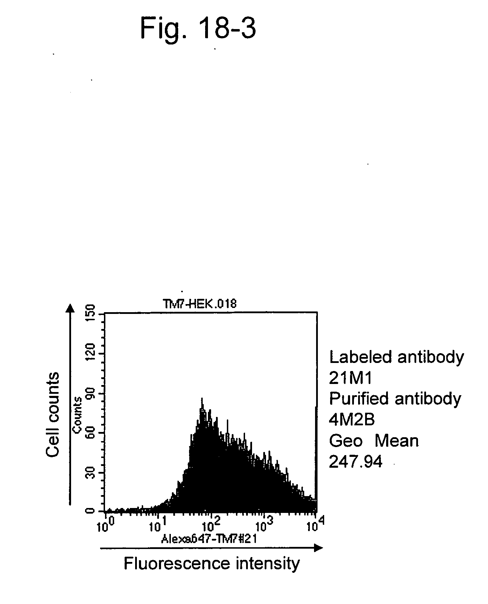

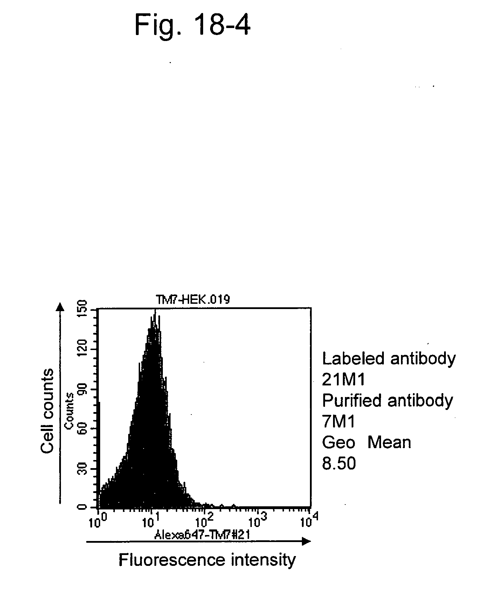

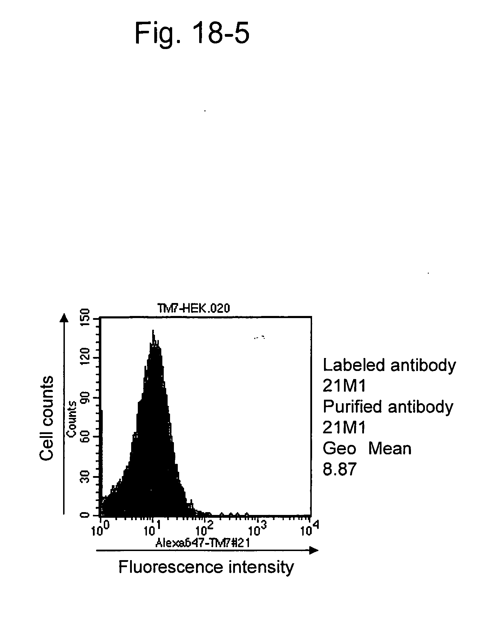

Fig. 18-1 shows competitiveness of 21M1 (labeled antibody), which is an anti-TM7XN1 monoclonal antibody, with KSA7, 4M2B, 7M1, or 21M1 (purified antibody). Fig. 18-1 also shows the binding amounts (fluorescence intensities) of 21M1 (labeled antibody) when a TM7XN1-EGFP fusion-protein-expressing cell line has been stained with 21M1 (labeled antibody) alone.
Fig. 18-2 shows competitiveness of 21M1 (labeled antibody), which is an anti-TM7XN1 monoclonal antibody, with KSA7, 4M2B, 7M1, or 21M1 (purified antibody). Fig. 18-2 also shows the binding amounts (fluorescence intensities) of 21M1 (labeled antibody) when a TM7XN1-EGFP fusion-protein-expressing cell line has been stained with 21M1 (labeled antibody) and KSA7 (purified antibody).
Fig. 18-3 shows competitiveness of 21M1 (labeled antibody), which is an anti-TM7XN 1 monoclonal antibody, with KSA7, 4M2B, 7M 1, or 21M 1 (purified antibody). Fig. 18-3 also shows the binding amounts (fluorescence intensities) of 21M1 (labeled antibody) when a TM7XN1-EGFP fusion-protein-expressing cell line has been stained with 21M1 (labeled antibody) and 4M2B (purified antibody).
Fig. 18-4 shows competitiveness of 21M1 (labeled antibody), which is an anti-TM7XN1 monoclonal antibody, with KSA7, 4M2B, 7M1, and 21M1 (purified antibody). Fig. 18-4 also shows the binding amounts (fluorescence intensities) of 21M 1 (labeled antibody) when a TM7XN1-EGFP fusion-protein-expressing cell line has been stained with 21M1 (labeled antibody) and 7M1 (purified antibody).
Fig. 18-5 shows competitiveness of 21M1 (labeled antibody), which is an anti-TM7XN 1 monoclonal antibody, with KSA7, 4M2B, 7M1, or 21M 1 (purified antibody). Fig. 18-5 also shows the binding amounts (fluorescence intensities) of 21M 1 (labeled antibody) when a TM7XN1-EGFP fusion-protein-expressing cell line has been stained with 21M 1 (labeled antibody) and 21M 1 (purified antibody).

### Best Mode for Carrying Out the Invention

Hereafter, the present invention is described in detail.

The present invention is based on a finding that an antibody against TM7XN1 significantly suppresses the metastasis of cancer cells expressing TM7XN1. In the present invention, a bone metastasis model and a spleen metastasis model established via orthotopic transplantation have been used. However, in view of a common mechanism shared by cancer cell metastasis cases, it is considered that metastasis not only to the bone marrow and the spleen, but also to other organs such as the lungs, the liver, and the brain, can also be effectively suppressed. TM7XN1 and GPR56 are almost completely identical to each other in terms of the extracellular region structure. Hence, it is considered that anti-TM7XN1 monoclonal antibodies (KSA7, 4M2B, 7M1, and 21M1) bind also to GPR56, which is expressed on cell surfaces. Moreover, there is an extremely high possibility that the expression sites of TM7XN1 and GPR56 or the cells expressing TM7XN1 and GPR56 have been reported without distinguishing between TM7XN1 and GPR56 (see Zendman AJ et al., (1999), FEBS Lett. 446: 292-298; and Liu M et al., (1999), Genomics 55: 296-305). Regarding descriptions concerning the expression site of TM7XN1 or GPR56 or the cells expressing TM7XN1 or GPR56 in these reports, there is also an extremely high possibility that such expression site of TM7XN1 can be the same as that of GPR56 or that the cells expressing TM7XN1 can be the same as those expressing GPR56. Therefore, a cell surface molecule to which KSA7, 4M2B, 7M1, or 21M1 binds can be not only TM7XN1, but also GPR56, regardless of expression sites and cells. Hence, there is an extremely high possibility that in the present invention, a novel finding obtained concerning TM7XN1 is also applicable to GPR56. Accordingly, examples of "an antibody against TM7XN1" of the present invention include not only antibodies against TM7XN1 such as anti-TM7XN1 monoclonal antibodies including KSA7, 4M2B, 7M1, and 21M1, but also antibodies against GPR56. Furthermore, another example of such antibody functions antagonistically against a ligand via its binding to TM7XN1. Examples of such antibody include an antibody that sterically blocks binding between TM7XN1 and a ligand and an antagonist antibody. "Antibody" in the present invention is derived from a gene (generically named "antibody gene") encoding a heavy chain variable region, a heavy chain constant region, a light chain variable region, and a light chain constant region composing an immunoglobulin. Examples of an "antibody" of the present invention also include functional fragments of such antibody. "Functional fragment" refers to a portion (partial fragment) of an antibody that possesses one or more effects (of the antibody) on an antigen. Specific examples of such functional fragment include F(ab')₂, Fab', Fab, Fv, disulfide bond Fv, single-chain Fv(scFv), and polymers thereof [D. J. King., Applications and Engineering of Monoclonal Antibodies., 1998 T. J. International Ltd]. Alternatively, "functional fragment" refers to a fragment of an antibody that is capable of binding to an antigen.

"Variant of an antibody" refers to a variant having an amino acid sequence derived from the amino acid sequence of an antibody produced by a hybridoma by deletion, substitution, insertion, or addition of 1 or more amino acids. A variant of the antibody of the present invention can be appropriately produced using a method for isolating an antibody gene from a hybridoma, sequence information concerning human antibody constant regions, a site-directed mutagenesis method for genes, and the like, which are known by persons skilled in the art.

In the present invention, an antibody can be obtained by incorporating an antibody gene into an expression vector, introducing the vector into an appropriate host cell, and then collecting and purifying the antibody from the cell or the culture supernatant of the cell.

The antibodies of the present invention can be produced by the following production methods, for example. Specifically, for example, a non-human mammal (e.g., a mouse, a rabbit, a goat, or a horse) is immunized with the above-defined TM7XN 1 or a part thereof, a product obtained by binding TM7XN 1 or a part thereof with an appropriate substance (e.g., bovine serum albumin) for enhancing the antigenicity of an antigen or cells expressing TM7XN1 in large amounts on cell surfaces, together with an immunostimulant (e.g., Freund's Adjuvant), if necessary. Alternatively, immunization can be conducted by administering an expression vector having TM7XN1 incorporated therein to such a non-human mammal. Monoclonal antibodies can be produced by preparing hybridomas from antibody-producing cells obtained from immunized animals and myeloma cells incapable of producing autoantibodies, cloning the hybridomas, and selecting clones that produce monoclonal antibodies showing specific affinity for the antigen used for immunization. More preferably, a non-human animal retaining a non-rearranged human antibody gene and producing a human antibody specific to the immunogen as a result of immunization is used for immunization, so that the antibody of the present invention can be obtained as a human antibody. An example of such non-human animal is a mouse. A method for producing mice capable of producing a human antibody is described in International Publication WO02/43478. Here, "human antibody" means an antibody that is an expression product of a human-derived antibody gene or a functional fragment thereof.

Furthermore, a recombinant antibody of the present invention can be prepared by gene recombination technology; specifically, by cloning a gene encoding a monoclonal antibody from antibody-producing cells such as hybridomas, incorporating the cloned gene into an appropriate vector, and then introducing the vector into a host (e.g., a mammalian cell line such as Chinese hamster ovary (CHO) cells, *Escherichia coli*, yeast cells, insect cells, and plant cells) (P. J. Delves., ANTIBODY PRODUCTION ESSENTIAL TECHNIQUES., 1997 WILEY, P. Shepherd and C. Dean., Monoclonal Antibodies., 2000 OXFORD UNIVERSITY PRESS; J. W. Goding., Monoclonal Antibodies: principles and practice., 1993 ACADEMIC PRESS). Moreover, transgenic cattle, goats, sheep, or pigs, wherein a target antibody gene is incorporated into the endogenous gene by transgenic animal production techniques, are generated. From the milk of these transgenic animals, monoclonal antibodies derived from the antibody gene can also be obtained in large quantities. When hybridomas are cultured *in vitro,* hybridomas are grown, maintained, and stored in a way suitable for various conditions such as the properties of cell species to be cultured, purposes of experiments and studies, and culturing methods. Then, hybridomas can be cultured using any nutrient medium that is induced and prepared from a known nutrient medium or known basic medium that is used for the production of monoclonal antibodies in the culture supernatant.

Examples of known cancer that frequently metastasizes to bone are as follows. Multiple myeloma, which is a hematogenous malignant tumor, setteles (homing) bone at a frequency of almost 100% and then grows while destroying the bone (Pearse RN et al., (2001), Proc Natl Acad Sci U.S.A. 98:11581-11586). Examples of known solid cancer that metastasizes to bone as a target organ for its metastasis include breast cancer, prostate cancer, lung cancer, thyroid gland cancer, gastric cancer, and malignant melanoma (Celeman (1997), Cancer 80: 1588-1594). Moreover, childhood neuroblastoma also frequently metastasizes to bone and is a major cause of poor prognosis (Michigami et al., (2001), Cancer Res 61: 1637-1644). In view of the examples of the present invention and the properties of cancer, it is considered that the antibodies of the present invention are effective against bone metastasis of at least TM7XN1-expressing multiple myeloma, breast cancer, prostate cancer, lung cancer, thyroid gland cancer, gastric cancer, malignant melanoma, and neuroblastoma.

Furthermore, from the view point of metastasis mechanism, a process whereby primary cancer enters blood vessels, flows in the blood stream, and reaches a target organ is common among all forms of metastasis to target organs. Antibodies shown in the present invention are likely to be also effective against cancer metastasis to the lungs, the liver, the brain, and the like. Therefore, the method of the present invention is also useful for prevention of the metastasis of various carcinomas including fibrosarcoma, myxosarcoma, liposarcoma, chondrosarcoma, osteogenic sarcoma, chordoma, angiosarcoma, endothelial sarcoma, lymphatic sarcoma, endothelial sarcoma of the lymphatic vessel, periosteal tumor, endothelioma, Ewing's tumor, leiomyosarcoma, rhabdomyoblastoma, carcinoma of colon, pancreatic carcinoma, breast cancer, ovarian cancer, prostate cancer, lymphoma, leukemia, carcinoma planoepitheliale, basal-cell carcinoma, adenosarcoma, sweat gland carcinoma, sebaceous gland carcinoma, papillary carcinoma, cystadenocarcinoma, medullary carcinoma, bronchial cancer, renal cell carcinoma, hepatic cancer, bile duct cancer, chorionic carcinoma, seminoma , embryonal carcinoma, Wilms' tumor, neck cancer, testicular tumor, lung cancer, small cell lung cancer, bladder cancer, epithelioma, glical cancer, spongiocytoma, medulloblastoma, craniopharyngioma, ependymoma, pinealoma, hemangioblastoma, acoustic nerve tumor, oligodendroglioma, meningioma, melanoma, neuroblastoma, and retinoblastoma, as long as TM7XN1 is expressed in these various carcinomas.

Furthermore, as described above, TM7XN1 and GPR56 are almost completely identical to each other in terms of the extracellular region structure. Hence, there is an extremely high possibility that anti-TM7XN1 monoclonal antibodies KSA7, 4M2B, 7M1, and 21M1 would also bind to GPR56 expressed on cell surfaces. Moreover, there is an extremely high possibility that the expression sites of TM7XN1 and GPR56 or the cells expressing TM7XN1 and GPR56 have been reported without distinguishing between TM7XN1 and GPR56 (see above Zendman AJ et al., (1999), FEBS Lett. 446: 292-298 and Liu M et al., (1999), Genomics 55: 296-305). Regarding description concerning the expression site of TM7XN1 or GPR56 or the cells expressing TM7XN1 or GPR56 in these reports, there is also an extremely high possibility that such expression site of TM7XN1 can be the same as that of GPR56 or that the cells expressing TM7XN 1 can be the same as those expressing GPR56. Therefore, a cell surface molecule to which KSA7, 4M2B, 7M1, or 21M1 binds can be not only TM7XN1 but also GPR56, regardless of expression sites and cells. Hence, there is an extremely high possibility that in the present invention, novel findings obtained concerning TM7XN1 are also applicable to GPR56. Accordingly, examples of "a cell of the cancer expresses TM7XN1 on its surface" of the present invention include not only cancer, the cells of which express TM7XN1 on the cancer cell surfaces, but also cancer, the cells of which express GPR56 on the same.

### Administration methods

The present invention is useful in treatment of medical conditions or diseases associated with cancer. The antibodies of the present invention can be combined with the other types of treatment (e.g., chemotherapy, irradiation therapy, immunotherapy, and surgical methods) for medical conditions as described in this description. The antibodies of the present invention can also be used in combination with agents including alkylating agents, antimetabolites, antihormones, and therapeutic drugs for various symptoms (e.g., analgesic agents, diuretic agents, antidiuretic agents, antiviral agents, antibiotics, nutritional supplements, anemia therapeutic drugs, therapeutic drugs against blood coagulation, therapeutic drugs for bone, psychiatric therapeutic drugs, and psychological therapeutic drugs).

For example, to prepare a pharmaceutical or sterile composition containing an antibody of the present invention, a material is mixed with, preferably, an inactive pharmaceutically acceptable carrier or excipient. Preparation of such pharmaceutical composition is known in the relevant field. Such therapeutic composition is typically sterile.

An antibody composition of the present invention is generally administered parenterally and is preferably administered into the vein. Such antibody composition can be immunogenic. Such antibody is thus slowly administered using, preferably, a conventional IV administration set or a subcutaneous reservoir to a dose ranging from 10 µg to 100 mg/kg/day.

When a therapeutic drug is administered parenterally, it is typically prescribed in the form of an injectable unit dose (solution, suspension, or emulsion) in combination with a pharmaceutically acceptable parenteral vehicle. Such vehicle is primarily avirulent and is nontherapeutic. An antibody can be administered in the form of a water-soluble vehicle such as water or a physiological saline solution or a buffered vehicle in combination with or without various additives and/or excipients. Alternatively, a suspension such as a zinc suspension can be prepared to contain an antibody. Such suspension can be useful for subcutaneous (SC), intradermal (ID), or intramuscular (IM) injection. The ratio of the entity of a therapeutic drug to an additive can vary widely, as long as both can exist in effective doses. Such a therapeutic drug is preferably in a purified form substantially containing no aggregates, other proteins, endotoxins, or the like. The drug is prescribed at a concentration between approximately 5 and 30 mg/ml, and preferably between 10 and 20 mg/ml. A preferable endotoxin level is less than 2.5 EU/ml.

Selection of medication regimens for an antibody for treatment depends on several factors (including serum or tissue cycling rates resulting from the use of a therapeutic drug, immunogenicity of a therapeutic drug, accessibility to target cells, and general patient tolerance for treatment stresses). Preferably, such medication regimens maximize the amount of a therapeutic drug to be delivered to a patient in view of side effects at acceptable levels. Therefore, the amount of a therapeutic drug to be delivered partially depends on the severity of conditions against which treatment is carried out.

An appropriate dose is determined using parameters or factors known in the relevant field, which affect or may affect treatment as predicted by clinicians. In general, administration is initiated with a dose that is somewhat lower than the optimum dose. Subsequently, the dose is gradually increased until a desired or optimum effect is achieved concerning any related side effects. The number of receptor-possessing cells in an identified sample can be an important index indicating achievement of the administration of an effective dose. Preferably, an antibody or an antibody-bound compound to be used herein is derived from an animal of the same species as that of a target animal for treatment, thereby minimizing humoral response to the reagent.

Regarding the weekly dose range of an antibody or an antibody-bound fragment, such weekly dose is approximately 10 µg, preferably approximately at least 50 µg, and more preferably at least approximately 100 µg or greater, per kg body weight. In general, such weekly dose is approximately less than 1000 µg, preferably approximately less than 500 µg, and more preferably approximately less than 100 µg, per kg body weight. A dose can be periodically administered for a shorter or longer time period based on a planned period for carrying out treatment as desired. Regarding a general dose range, such dose ranges from at least approximately 10 µg to approximately 50 µg, and preferably from approximately 100 µg to approximately 10 mg, per kg body weight.

"Effective dose" refers to an amount sufficient for causing a desired response, alleviating the symptoms of or taking measures against the signs of metastases or primary tumor advancement, diminishing tumor sizes, or alleviating tumor growth. Examples of typical mammals that can be treated using a composition containing an antibody of the present invention include mice, rats, cats, dogs, and primates (including humans). The effective dose for a specific patient can be varied depending on factors including conditions against which treatment is carried out, the patient's overall health conditions, the method and the route for administration, the dose, and the severity of side effects. The effects of such dose are expected to be able to cause a quantifiable change by at least approximately 10%, and preferably by at least approximately 20%, 30%, 50%, 70%, 90%, or more. When several components are used in combination, an effective dose constitutes a percentage of the total combination of components. The effects are not limited to those of each component.

The effective dose of a therapeutic drug typically regulates symptoms by at least approximately 10%, generally at least approximately 20%, preferably at least approximately 30%, and more preferably at least approximately 50%. Alternatively, cell transfer regulation refers to affect the transfer of each of various cell types or information exchange. Cell transfer regulation causes a statistically significant and quantifiable change in the number of affected cells, for example. Such a change can be a decrease in the number of target cells that are attracted within a predetermined duration or into a target region. The speed of primary tumor advancement, primary tumor size, or primary tumor growth can also be monitored.

Furthermore, as therapeutic drugs to which the present invention is applied, antisense nucleic acids can be used in place of antibodies. For example, an antisense polynucleotide against TM7XN1 can specifically suppress TM7XN1 gene expression through introduction of the antisense polynucleotide into cells. Such antisense polynucleotide can function in a manner similar to that of a receptor antagonist. Thus, a target therapeutic effect can be exerted.

Moreover, as therapeutic drugs to which the present invention is applied, RNAi (RNA interference) methods can be used in place of antibodies. RNAi is a phenomenon whereby gene expression is suppressed in a sequence-specific manner by a 21- to 23-nucleotide double-stranded RNA referred to as siRNA (short interfering RNA) (Fire A et al., (1998), Nature 391: 806-811, Elbashir SM et al., (2001), Nature 411: 494-498). Among such RNAi methods, methods for introducing siRNA into cells can be classified into methods that involve introducing synthetic siRNA into cells and methods that involve introducing an siRNA expression vector into cells and causing the expression of a double-stranded RNA. Hence, for example, it becomes possible for synthetic siRNA or an siRNA expression vector, the sequence of which is identical to the TM7XN1 gene sequence to function in a manner similar to that of a TM7XN1 gene receptor antagonist through its introduction into cells. Therefore, a target therapeutic effect can be exerted.

### Examples

The present invention is hereafter described in greater detail with reference to the following examples, although the present invention is not limited thereto.

### Example 1: Metastasis-suppressing effect of anti-TM7XN1 monoclonal antibody KSA7 in bone metastasis model

Experimental materials A human acute myeloid leukemia cell line (KG1a) was purchased from Dainippon Pharmaceutical Co., Ltd. and then cultured under conditions of IMDM + 10% FBS, 37°C, and 5% CO₂. 5-week-old male immunodeficient mice (NOD/SCID/γ_{c} null mice: commonly known as NOG mice) were purchased from the Central Institute for Experimental Animals (CIEA). These mice were used for a transplantation experiment when they were 14-week-old. KSA7 is a monoclonal antibody (rat IgG2b) obtained by immunizing rats with a TM7XN1-forcibly-expressing 3Y1 rat cell line at the Pharmaceutical Research Laboratories, Pharmaceutical Division, KIRIN Brewery Co., Ltd. KSA7 was prepared in the form of a 5mg/mL PBS solution, stored, and then used.

### Experimental methods

KG1a cells were cultured at a cell density ranging from 1.0 × 10⁵ to 5.0 × 10⁵ cells/mL. 3 NOG mice each were used for a control group and a KSA7-administered group. For transplantation into the control group, a cell suspension was prepared at a concentration of 3.6 × 10⁶ KG1 a cells/600 µL using IMDM + 0.1 % BSA. For transplantation into the antibody-administered group, a cell suspension was prepared at a concentration of 3.6 × 10⁶ KG1a cells + KSA7 45 µg/600 µL using a PBS solution containing IMDM + 0.1% BSA and 5 mg/mL KSA7. Each cell suspension was allowed to stand at 4°C for 60 minutes. 200 µL (per mouse) of the cell suspension prepared for the control group or the antibody-administered group was transplanted via the tail vein into NOG mice that had been exposed to 200 cGy X-ray before transplantation. 2 weeks after transplantation, bone marrow cells in the thigh bone were collected from the mice of each group. The cells were subjected to double staining using an anti-mouse CD45 PE labeled antibody and an anti-human CD45 APC labeled antibody. The number of mouse CD45-positive cells and the number of human CD45-positive cells contained in a predetermined number of viable cells were measured by a flow cytometry method. Then, % of chimerism was calculated based on a calculating formula: the number of human CD45-positive cells ÷ (number of mouse CD45-positive cells + number of human CD45-positive cells) × 100 (%). High % of chimerism indicates a high survival rate of KG1a cells (human CD45-positive cells) in the bone marrow. Specifically, high % of chimerism indicates that many of these cells had metastasized to the bone marrow.

### Results

Average % of chimerism (± standard deviation) of the control group was 10.42% (± 6.49) and average % of chimerism of the KSA7-administered group was 2.69% (+1.22)(Fig. 1). Z test was then carried out using a significance level of 0.05, resulting in p = 0.02. This result indicated that bone marrow metastasis of KG1a cells had been significantly suppressed by treatment with KSA7, compared with the control group. Furthermore, it was suggested by the result that the anti-TM7XN1 monoclonal antibody KSA7 can suppress the bone marrow metastasis of cancer cells expressing TM7XN1.

### Example 2: Activity of anti-TM7XN1 monoclonal antibody KSA7 to bind to TM7XN1 Experimental materials

An HEK293 cell line (derived from human embryonic kidney cells) was cultured under conditions of DMEM + 10% FBS, 37°C, and 5% CO₂. An HEK293 cell line forcibly expressing a TM7XN1-EGFP fusion-protein was established at the Pharmaceutical Research Laboratories, Pharmaceutical Division, KIRIN Brewery Co., Ltd. and then cultured under conditions of DMEM + 10% FBS + 5 µg/mL Blasticidin, 37°C, and 5% CO₂. KSA7 is a monoclonal antibody (rat IgG2b) obtained by immunizing rats with a 3Y1 rat cell line forcibly expressing TM7XN1 at the Pharmaceutical Research Laboratories, Pharmaceutical Division, KIRIN Brewery Co., Ltd. KSA7 was prepared in the form of a 500 µg/mL PBS solution and then used. As an isotype antibody (rat IgG2b, 500 µg/mL), BD-553986 (Becton-Dickinson Japan) was used. As a secondary antibody (PE-labeled anti-rat IgG antibody F(ab')2 fragment), OBMSTAR73 (COSMO BIO Co., Ltd.) was used. FACSCalibur4A (Becton-Dickinson Japan) was used as a flowcytometer. A staining medium was prepared by adding 2% (v/v) FBS, 5 mM EDTA-2Na, and 0.05% (w/v) NaN₃ (all of these concentrations were final concentrations) to PBS. PI + staining medium was prepared by adding propidium iodide to the staining medium at 1 µg/mL (final concentration).

### Experimental methods

The HEK293 cell line (hereinafter, referred to as the control cell line) and the HEK293 cell line forcibly expressing the TM7XN1-EGFP fusion protein (hereinafter, referred to as the expression cell line) were removed from culture plates. Each cell line was prepared at a cell concentration of 1.0 × 10⁷ cells/mL in the staining medium. 50 µL each of the staining medium was dispensed into a 1.5 mL tube. KSA7 or the isotype antibody was added to each tube to a final concentration of 50 µg/mL. After each resultant was allowed to stand at 4°C for 30 minutes, 1 mL of the staining medium was added. Centrifugation was performed at 400 G for 3 minutes to remove the supernatant and then the cells were washed. An undiluted solution of the secondary antibody was diluted to 1/50 of the total volume using the staining medium. 50 µL of the diluted solution was added to the thus washed cells. After each resultant was allowed to stand at 4°C for 30 minutes, 1 mL of the staining medium was added. Centrifugation was performed at 400 G for 3 minutes to remove the supernatant and then the cells were washed. 400 µL of the PI + staining medium was added to each resultant so that the cells were suspended. Binding of antibodies to the cells was determined using a flowcytometer. The expression levels of the TM7XN1-EGFP fusion protein and the like were measured using fluorescence intensity.

### Results

KSA7 was added to the control cell line and the expression cell line and then the resulting fluorescence intensity of the control cell line was compared with the same of the expression cell line. As shown in Fig. 2, it was revealed that KSA7 binds only to the expression cell line.

Furthermore, the isotype antibody and KSA7 were each separately added to the expression cell line. The expression cell line with the isotype antibody bound thereto was then compared with the expression cell line with KSA7 bound thereto. As shown in Fig. 3, it was revealed that KSA7 alone binds to the expression cell line. Moreover, the isotype antibody and KSA7 were each separately added to the expression cell line. Correlation between the expression level of the TM7XN1-EGFP fusion protein as an antigen and the binding amount of KSA7 was observed. As shown in Fig. 4 (in the case of the isotype antibody) and Fig. 5 (in the case of KSA7), it was revealed that KSA7 had been bound in accordance with the expression level of the antigen. Based on the above result, it was revealed that KSA7 specifically binds to TM7XN1.

### Example 3: Metastasis-suppressing effect of each of 4 types of anti-TM7XN1 monoclonal antibody (KSA7, 4M2B, 7M1, and 21M1) in bone metastasis model established using human acute myeloid leukemia cell line

### Experimental materials

A human acute myeloid leukemia cell line (KG1a) was purchased from Dainippon Pharmaceutical Co., Ltd. and then cultured under conditions of IMDM + 10% FBS, 37°C, and 5% CO₂. 6-week-old male NOD/Shi-*scid*, IL-2Rγ^{null} mice (NOG mice), the immunodeficient mice, were purchased from the Central Institute for Experimental Animals (CIEA). These mice were used for a transplantation experiment when they were 11-week-old. KSA7 is an anti-TM7XN1 monoclonal antibody (rat IgG2b) obtained by immunizing rats with a 3Y1 rat cell line forcibly expressing TM7XN1 at the Pharmaceutical Research Laboratories, Pharmaceutical Division, KIRIN Brewery Co., Ltd. KSA7 was prepared in the form of a 5 mg/mL PBS solution, stored, and then used. Furthermore, 4M2B, 7M1, and 21M1 were anti-TM7XN1 monoclonal antibodies (4M2B: mouse IgG2b; and 7M1 and 21M1: mouse IgG1) obtained via commissioned production by Immuno-Biological Laboratories Co., Ltd. Specifically, these antibodies were obtained by immunizing mice with a Balb3T3 mouse cell line forcibly expressing TM7XN1. Each of these antibodies was prepared in the form of a 2 mg/mL PBS solution, stored, and then used. Regarding isotype control antibodies, the products of Becton-Dickinson Japan including rat IgG2b (BD-553986, clone A95-1), mouse IgG2b (BD-559530, clone MPC-11), and mouse IgG1 (BD-557273, clone MOPC-31C) were purchased. Sodium azide was removed using a NAP-25 column (Amersham Biosciences) and then concentrated via ultrafiltration. Each resultant was prepared in the form of a 2 mg/mL PBS solution, stored, and then used.

Moreover, 21M1 was labeled with Alexa Fluor 647 using an Alexa Fluor 647 Protein Labeling Kit (Molecular Probes), prepared in the form of a 0.5 mg/mL PBS solution (supplemented with 0.05%(w/v) NaN₃), and then used. As a labeled isotype control antibody corresponding to labeled 21M1, a mouse IgG1-Alexa647 label (BD-557732, clone MOPC-21, Becton-Dickinson Japan) was purchased and then used. As an anti-human CD45 APC labeled antibody, IM2473 (Beckman Coulter, Inc.) was used. As an anti-mouse CD45 PE labeled antibody, BD-553081 (Becton-Dickinson Japan) was used. A staining medium was prepared by adding 2% (v/v) FBS, 5mM EDTA-2Na, 0.05% (w/v) NaN₃ (all of these concentrations were final concentrations) to PBS. PI + staining medium was prepared by adding propidium iodide to the staining medium at 1 µg/mL (final concentration). FACSCalibur4A (Becton-Dickinson Japan) was used as a flowcytometer.

### Experimental methods

KG1a cells were cultured at a cell density ranging from 1.0 × 10⁵ to 5.0 × 10⁵ cells/mL.

### (Confirmation of TM7XN1 expression in KG1a cells)

When KG1a cells in a state of being cultured were harvested for transplantation, some KG1a cells were isolated. The cells were prepared at a cell concentration of 1.0 × 10⁷ cells/mL using the staining medium and then 50 µL each of the resultant was dispensed in a 1.5 mL tube. Labeled 21M1 or each of the labeled isotype antibodies was added to the tube in an amount 1/10 (5.0 µL) of the total volume. After each resultant was allowed to stand at 4°C for 30 minutes, 1 mL of the staining medium was added. Centrifugation was performed at 400 G for 3 minutes to remove the supernatant and then the cells were washed. 350 µL of the PI + staining medium was added to each resultant so that the cells were suspended. The amount of an antibody binding to a surface antigen in viable cells was measured using a flowcytometer. Specifically, the expression levels of TM7XN1 were measured using fluorescence intensity.

### (Transplantation of KG1a cells)

6 NOG mice were used per group (a total of 48 mice) in a PBS-administered group, a rat IgG2b-administered group, a mouse IgG1-administered group, a mouse IgG2b-administered group, a KSA7-administered group, a 4M2B-administered group, a 7M1-administered group, and a 21M1-administered group. First, a necessary amount of a cell suspension containing KG1a cells was prepared at 1.0 × 10⁷ cells/mL using IMDM + 0.1% BSA. Next, for transplantation to the PBS-administered group, 1400 µL of the cell suspension supplemented with 175 µL of PBS was prepared. For transplantation into the rat IgG2b-administered group, the mouse IgG1-administered group, or the mouse IgG2b-administered group, 1400 µL of the cell suspension supplemented with 175 µL of each isotype control antibody solution was prepared. For transplantation to the KSA7-administered group, 1400 µL of the cell suspension supplemented with 70 µL of a KSA7 solution and 105 µL of PBS was prepared. For transplantation to the 4M2B-administered group, the 7M1-administered group, or the 21M1-administered group, 1400 µL of the cell suspension supplemented with 175 µL of each anti-TM7XN1 monoclonal antibody solution was prepared.

Each cell suspension was allowed to stand at 4°C for 60 minutes until the start of transplantation. 225 µL of the cell suspension (containing KG1a cells: 2.0 × 10⁶, antibody: 50 µg (in the case of the antibody-administered groups)) prepared for each antibody-administered group was transplanted via the tail vein into each of the NOG mice that had been exposed to 200 cGy X-ray before transplantation. 2 weeks after transplantation, bone marrow cells in the thigh bone were collected from the mice of each group. The cells were subjected to double staining using an anti-mouse CD45 PE labeled antibody and an anti-human CD45 APC labeled antibody. The number of mouse CD45-positive cells and the number of human CD45-positive cells contained in a predetermined number of viable cells were measured by a flow cytometry method. Then, % of chimerism was calculated based on a calculating formula: the number of human CD45-positive cells ÷ (number of mouse CD45-positive cells + number of human CD45-positive cells) × 100(%). High % of chimerism indicates a high engraftment rate of KG1a cells (human CD45-positive cells) in the bone marrow. Specifically, high % of chimerism indicates that many of these cells had metastasized to the bone marrow.

### Results

Each isotype control antibody was added to KG1a cells used for transplantation and 21M1 was added to the same and then the binding amounts (fluorescence intensities) of each antibody were measured. As shown in Fig. 6, compared with the case where the isotype control antibodies were added (Fig. 6-1), the binding amounts (fluorescence intensities) of the antibodies had clearly increased in the case where 21M1 was added (Fig. 6-2). Thus it could be confirmed that KG1a cells used for transplantation had expressed TM7XN1.

Meanwhile, average % of chimerism (± standard deviation) in the bone marrow of each antibody- (or PBS-) administered group at 2 weeks after transplantation is as follows. The average % of chimerism of the PBS-administered group was 2.69% (± 0.71), that of the rat IgG2b-administered group was 2.89% (± 0.93), that of the mouse IgG1-administered group was 1.58% (± 0.39), that of the mouse IgG2b-administered group was 2.86 % (± 1.55), that of the KSA7-administered group was 0.26% (± 0.15), that of the 4M2B-administered group was 0.09% (± 0.09), that of the 7M1-administered group was 0.37% (± 0.14), and that of the 21M1-administered group was 0.05% (± 0.03) (Fig. 7). Of these groups, the anti-TM7XN1 monoclonal antibody-administered group and the corresponding isotype control antibody-administered group were subjected to t-test (one-sided test) with a significance level of p < 0.01. The results were: p = 0.0002 in the case of the KSA7-administered group and the rat IgG2b-administered group; p = 0.0007 in the case of the 4M2B-administered group and the mouse IgG2b-administered group; p = 0.001 in the case of the 7M1-administered group and the mouse IgG1-administered group; and p = 0.0002 in the case of the 21M1-administered group and the mouse IgG1-administered group. Significant differences were shown in all the cases. It was demonstrated by these results that bone marrow metastasis of KG1a cells had been significantly suppressed by administration of the anti-TM7XN1 monoclonal antibody. It was also demonstrated by these results that not only a special single clone but also monoclonal antibodies against the TM7XN1 molecule can suppress the bone marrow metastasis of cancer cells expressing TM7XN1. It was thus demonstrated that the TM7XN1 molecule is an important target molecule for suppression of cancer cell metastasis.

### Example 4: The metastasis-suppressing effect of anti-TM7XN1 monoclonal antibody 21M1 in bone marrow-spleen metastasis model established using human multiple myeloma cell line

### Experimental materials

A human multiple myeloma cell line (RPMI8226) was purchased from JCRB (Japanese Collection of Research Bioresearches) and then cultured under conditions of RPMI 1640 + 10% FBS, 37°C, and 5% CO₂. 6-week-old male NOD/Shi-*scid*, IL-2Rγ^{null} mice (NOG mice), the immunodeficient mice, were purchased from the Central Institute for Experimental Animals (CIEA). They were used for a transplantation experiment when they were 19-week-old. 21M1 is anti-TM7XN1 monoclonal antibody (mouse IgG1) obtained via commissioned production by Immuno-Biological Laboratories Co., Ltd. Specifically, 21MI was obtained by immunizing mice with a Balb3T3 mouse cell line forcibly expressing TM7XN1. 21M1 was prepared in the form of a 2 mg/mL PBS solution, stored, and then used. Furthermore, 21M1 was labeled with Alexa Fluor 647 using an Alexa Fluor 647 Protein Labeling Kit (Molecular Probes). The resultant was prepared in the form of a 0.5 mg/mL PBS solution (supplemented with 0.05% (w/v) NaN₃) and then used. As a labeled isotype control antibody corresponding to labeled 21M1, a mouse IgG1-Alexa 647 label (BD-557732, clone MOPC-21, Becton-Dickinson Japan) was purchased and used. As a human HLA-ABC FITC labeled antibody, BD-555552 (Becton-Dickinson Japan) was used. As an anti-mouse CD45 PE labeled antibody, BD-553081 (Becton-Dickinson Japan) was used. A staining medium was prepared by adding 2% (v/v) FBS, 5 mM EDTA-2Na, and 0.05% (w/v) NaN₃ (all of these concentrations were final concentrations) to PBS. PI + staining medium was prepared by adding propidium iodide to the staining medium at 1 µg/mL (final concentration). FACSCalibur4A (Becton-Dickinson Japan) was used as a flowcytometer.

### Experimental methods

RPMI8226 cells were cultured at a cell density ranging from 1.0 × 10⁵ to 5.0 × 10⁵ cells/mL.

### (Confirmation of TM7XN1 expression in RPMI8226 cells)

When RPMI8226 cells in a state of being cultured were harvested for transplantation, some RPMI8226 cells were isolated. The cells were prepared at a cell concentration of 1.0 × 10⁷ cells/mL using the staining medium and then 50 µL each of the resultant was dispensed in a 1.5 mL tube. Labeled 21M1 or the labeled isotype antibody was added to the tube in an amount 1/10 (5.0 µL) of the total volume. After each resultant was allowed to stand at 4°C for 30 minutes, 1 mL of the staining medium was added. Centrifugation was performed at 400 G for 3 minutes to remove the supernatant and then the cells were washed. 350 µL of the PI + staining medium was added to each resultant so that the cells were suspended. The amount of an antibody binding to a surface antigen in viable cells was measured using a flowcytometer. Specifically, the expression levels of the TM7XN1 were measured using fluorescence intensity.

### (Transplantation of RPMI8226 cells)

6 NOG mice were used per group (a total of 12 mice) of a PBS-administered group (a total of 1 group) and a 21M1-administered group (a total of 1 group). First, a necessary amount of a cell suspension containing RPMI8226 cells at 2.0 × 10⁷ cells/mL was prepared using IMDM + 0.1% BSA. Next, for transplantation to the PBS-administered group, 1300 µL of the cell suspension supplemented with 162.5 µL of PBS was prepared. For transplantation into the 21M1-administered group, 1300 µL of the cell suspension supplemented with 162.5 µL of the antibody solution was prepared.

Each cell suspension was allowed to stand at 4°C for 60 minutes until the start of transplantation. 225 µL of the cell suspension (containing RPMI8226 cells: 4.0 × 10⁶, 21M1: 50 µg (in the case of the 21M1-administered group)) prepared for each group was transplanted via the tail vein into each of NOG mice that had been exposed to 200 cGy X-ray before transplantation. 4 weeks after transplantation, bone marrow cells in the thigh bone and spleen cells were collected from the mice of each group. The cells were subjected to double staining using an anti-mouse CD45 PE labeled antibody and an anti-human HLA-ABC FITC labeled antibody. The number of mouse CD45-positive cells and the number of human HLA-ABC-positive cells contained in a predetermined number of viable cells were measured by a flow cytometry method. Then, % of chimerism was calculated based on a calculating formula: the number of human HLA-ABC-positive cells ÷ (number of mouse CD45-positive cells + number of human HLA-ABC-positive cells) × 100(%). High % of chimerism indicates a high engraftment rate of RPMI8226 cells (human HLA-ABC-positive cells) in the bone marrow or the spleen. Specifically, high % of chimerism indicates that many of these cells had metastasized to the bone marrow or the spleen.

### Results

The isotype control antibody and 21M1 were each separately added to RPMI8226 cells used for transplantation and then the binding amounts (fluorescence intensities) of the antibodies were measured. As shown in Fig. 8-1 and Fig. 8-2, compared with the case in which the isotype control antibody was added (Fig. 8-1), the binding amounts (fluorescence intensities) of 21M1 had clearly increased (Fig. 8-2). Thus it could be confirmed that RPMI8226 cells used for transplantation had expressed TM7XN 1.

Meanwhile, average % of chimerism (± standard deviation) in the bone marrow of each antibody- (or PBS-) administered group at 4 weeks after transplantation is as follows. The average % of chimerism of the PBS-administered group was 0.11% (± 0.07) and that of the 21M1-administered group was 0.08 % (± 0.07) (Fig. 9-1). Furthermore, average % of chimerism (± standard deviation) in the spleen of each antibody- or PBS-administered group is as follows. The average % of chimerism of the PBS-administered group was 4.55% (± 1.72) and that of the 21M1-administered group was 3.36 % (± 2.49) (Fig. 9-2). It was revealed by these results that there is a tendency of the anti-TM7XN1 monoclonal antibody to suppress the bone marrow metastasis and the spleen metastasis of RPMI8226 cells. It was further demonstrated by these results that monoclonal antibodies against the TM7XN1 molecule can suppress the bone marrow metastasis and the spleen metastasis of cancer cells expressing TM7XN1. Therefore, it was demonstrated that the TM7XN1 molecule is an important target molecule for suppressing cancer cell metastasis.

### Example 5: The metastasis-suppressing effect of anti-TM7XN1 monoclonal antibody 21M1 in model for visual detection of metastasis following orthotopic transplantation of solid cancer cells

### Experimental materials

A human pancreatic cancer cell line BxPC3 (hereinafter, referred to as the cancer cell line) expressing TM7XN1 and forcibly expressing GFP was established, cultured and transplanted at ANTICANCER, INC. (Sandiego, CA, U.S.A.). As animals to be used for transplantation of the cancer cell line, 5- to 6-week-old male nude mice (NCr nu/nu) were used. 21M1 is an anti-TM7XN1 monoclonal antibody (mouse IgG1) obtained via commissioned production by Immuno-Biological Laboratories Co., Ltd. Specifically, 21M1 was obtained by immunizing the mice with a Balb3T3 mouse cell line forcibly expressing TM7XN1. 21M1 was prepared in the form of a 2 mg/mL PBS solution, stored, and then used. As an isotype control antibody, mouse IgG1 (BD-557273, clone MOPC-31C, Becton-Dickinson Japan) was purchased.
Sodium azide was removed using a NAP-25 column (Amersham Biosciences) and then concentrated via ultrafiltration. The resultant was prepared in the form of a 2 mg/mL PBS solution, stored, and then used. Furthermore, 21M1 was labeled with Alexa Fluor 647 using an Alexa Fluor 647 Protein Labeling Kit (Molecular Probes). The resultant was prepared in the form of a 0.5 mg/mL PBS solution (supplemented with 0.05% (w/v) NaN₃). As a labeled isotype control antibody corresponding to labeled 21M1, a mouse IgG1-Alexa 647 label (BD-557732, clone MOPC-21, Becton-Dickinson Japan) was purchased and then used. A staining medium was prepared by adding 2% (v/v) FBS, 5 mM EDTA-2Na, and 0.05% (w/v) NaN₃ (all of these concentrations were final concentrations) to PBS. PI + staining medium was prepared by adding propidium iodide to the staining medium at 1 µg/mL (final concentration). An LZ12 stereoscopic fluorescence microscope (Leica) equipped with a mercury lamp was used for GFP imaging. A D425/60 band-pass filter and a 470 DCXR dichroic mirror (Chroma Technology Corporation) were used for GFP excitation. Furthermore, emitted GFP fluorescence was imported as images using a GG475 long-pass filter (Chroma Technology Corporation) and Hamamatsu C5810 3-chip cooled color CCD camera (Hamamatsu Photonics K.K.). The thus imported images were processed and analyzed using Image Pro Plus 3.1 software (Media Cybernetics, Inc.).

### Experimental methods

### (Confirmation of TM7XN1 expression in cancer cell lines)

The cancer cell line cultured while adhering to each culture plate was removed and harvested via several minutes of treatment using a 0.05% trypsin solution from the culture plate. The staining medium was added in an equivalent amount to inactivate trypsin. Centrifugation was performed at 400G for 3 minutes and then the cells were washed. The staining medium was added to the resultant so that the cells were suspended. The resultant was prepared to a cell concentration of approximately 2.5 × 10⁷/mL and then 20 µL each of the resultant was dispensed to a 1.5 mL tube. Labeled 21M1 or the labeled isotype antibody was added to the tube in an amount 1/10 (2.0 µL) of the total volume. After each resultant was allowed to stand at 4°C for 30 minutes, the cells were fixed with a 4% paraformaldehyde/PBS solution added thereto. Centrifugation was performed at 400 G for 3 minutes to remove the supernatant. 350 µL of the staining medium was added to each resultant so that the cells were suspended. The amount of an antibody binding to a surface antigen of the cancer cell line was measured using a flowcytometer. Specifically, the expression levels of the TM7XN1 were measured using fluorescence intensity.

### (Transplantation of cancer cell lines)

A cancer cell line mass that had been maintained and subjected to subculture in nude mice for cancer cell line stock was cut into pieces each the size of 1 mm³. The abdominal cavity of each nude mouse for transplantation was opened under anesthesia. Three to four 1-mm³-cancer cell line portions were transplanted into the pancreas of each nude mouse for transplantation. The opened abdominal cavity was then sutured. About 4 weeks after transplantation, nude mice, for which survival of the cancer cell line had been confirmed via manipulation, were selected. The mice were grouped into a PBS (medium)-administered group, a 21M1 (3 mg/kg)-administered group, and a mouse IgG1 (isotype control antibody) 3 mg/kg-administered group (a total of 3 groups, 10 mice per group, and a total of 30 mice). Immediately after confirmation of survival of the cells and grouping, each antibody (or PBS) was administered to the mice of each group in the relevant amount via the tail vein. Subsequently, each antibody (or PBS) was administered weekly. At 5 weeks after the start of administration, the abdominal cavity of each mouse was opened, GFP fluorescence was imported as images, and then the presence or the absence of metastatic tumor was determined. Furthermore, the weights of primary lesions caused to survive in the pancreas were also measured. Results

Each isotype control antibody and 21M1 were each separately added to the cancer cell line and then the binding amounts (fluorescence intensities) of each antibody were measured. As shown in Fig. 10, compared with the case in which the isotype control antibody was added (Fig. 10-1), the binding amounts (fluorescence intensities) of 21M1 had clearly increased (Fig. 10-2). Thus it could be confirmed that the cancer cell line had expressed TM7XN 1.

Meanwhile, one mouse of the PBS-administered group had died before opening of the abdominal cavity and analysis of transplanted nude mice. Therefore, opening of the abdominal cavity for the analysis was performed for the PBS-administered group (9 mice), the 21M1 (3 mg/kg)-administered group (10 mice), and the mouse IgG1 (3 mg/kg)-administered group (10 mice). Upon opening of the abdominal cavity, the presence or the absence of metastatic tumor in the spleen was determined and then the incidences of metastasis to the spleen were compared. The incidence of the PBS-administered group was 66.7% (6 out of 9 mice), the same of the 21M1 (3 mg/kg)-administered group was 30.0% (3 out of 10 mice), and the same of the mouse IgG1 (3 mg/kg)-administered group was 80.0% (8 out of 10 mice) (Fig. 11-2). Hence, it can be said that the incidence of spleen metastasis tended to be decreased by the administration of 21M1.

Based on the above results, it was demonstrated that monoclonal antibodies against the TM7XN1 molecule can suppress spleen metastasis of pancreatic cancer cells (BxPC3-3) expressing TM7XN1.

### Example 6: Specificity of the binding of anti-TM7XN1 monoclonal antibodies KSA7, 4M2B, 7M 1, and 21M1 to TM7XN1

### Experimental materials

An HEK293 cell line (derived from human embryonic kidney cells) was cultured under conditions of DMEM + 10% FBS, 37°C, and 5% CO₂. An HEK293 cell line forcibly expressing an EGFP protein and an HEK293 cell line forcibly expressing a TM7XN1-EGFP fusion protein were established at the Pharmaceutical Research Laboratories, Pharmaceutical Division, KIRIN Brewery Co., Ltd. The cell lines were cultured under conditions of DMEM + 10% FBS + 5 µg/mL Blasticidin, 37°C, and 5% CO₂. KSA7 is an anti-TM7XN1 monoclonal antibody (rat IgG2b) obtained by immunizing rats with a 3Y1 rat cell line forcibly expressing TM7XN1 at the Pharmaceutical Research Laboratories, Pharmaceutical Division, KIRIN Brewery Co., Ltd. KSA7 was labeled with Alexa Fluor 647 using an Alexa Fluor 647 Protein Labeling Kit (Molecular Probes). Labeled KSA7 was then prepared in the form of a 0.5 mg/mL PBS solution (supplemented with 0.05% (w/v) NaN₃) and then used. 4M2B, 7M1, and 21M1 were anti-TM7XN1 monoclonal antibodies (4M2B: mouse IgG2b; and 7M1 and 21M1: mouse IgG1) obtained via commissioned production by Immuno-Biological Laboratories Co., Ltd. Specifically, these antibodies were obtained by immunizing mice with a Balb3T3 mouse cell line forcibly expressing TM7XN1. Each of 4M2B, 7M1, and 21M1 was labeled with Alexa Fluor 647 using an Alexa Fluor 647 Protein Labeling Kit (Molecular Probes), prepared in the form of a 0.5 mg/mL PBS solution (supplemented with 0.05% (w/v) NaN₃), and then used. Regarding isotype control antibodies, the products of Becton-Dickinson Japan including rat IgG2b (BD-553986, clone A95-1), mouse IgG2b-Alexa647 label (BD-557903, clone 27-35), mouse IgG1-Alexa647 label (BD-557732, clone MOPC-21) were purchased and then used. In addition, rat IgG2b was labeled with Alexa Fluor 647 using Alexa Fluor 647 Protein Labeling Kit (Molecular Probes), prepared in the form of a 0.5 mg/mL PBS solution (supplemented with 0.05% (w/v) NaN₃), and then used. As a flowcytometer, FACSCalibur4A (Becton-Dickinson Japan) was used. A staining medium was prepared by adding 2% (v/v) FBS, 5 mM EDTA-2Na, and 0.05% (w/v) NaN₃ (all of these concentrations were final concentrations) to PBS. PI + staining medium was prepared by adding propidium iodide to the staining medium at a concentration of 1 µg/mL (final concentration).

### Experimental methods

The HEK293 cell line forcibly expressing EGFP (hereinafter, referred to as the EGFP-expressing control cell line) and the HEK293 cell line forcibly expressing the TM7XN1-EGFP fusion protein (hereinafter, referred to as the TM7XN1-expressing cell line) were removed from the culture plates. The cells were prepared at a cell concentration of 1.0 × 10⁷ cells/mL using the staining medium. 25 µL each of the resultant was dispensed into a 1.5 mL tube. KSA7, 4M2B, 7M1, 21M1, or each isotype antibody was added to the tube in an amount 1/10 (2.5 µL) of the total volume. After each resultant was allowed to stand at 4°C for 30 minutes, 1 mL of the staining medium was added. Centrifugation was performed at 400 G for 3 minutes to remove the supernatant and then the cells were washed. 350 µL of the PI + staining medium was added to each resultant so that the cells were suspended. The binding amounts of antibodies to surface antigens, the expression levels of EGFP, and the expression levels of the TM7XN1-EGFP fusion protein in viable cells were measured using a flowcytometer and fluorescence intensity.

### Results

KSA7, 4M2B, 7M1, and 21M1 were each added to the TM7XN1-expressing cell line. Then the relationship between the expression levels of the TM7XN1-EGFP fusion protein and the binding amounts of the antibodies was studied. As revealed in Fig. 12, KSA7 (Fig. 12-1), 4M2B (Fig. 12-2), 7M1 (Fig. 12-3), and 21M1 (Fig. 12-4) were each bound in a manner depending on the expression level of the TM7XN1-EGFP fusion protein.

Moreover, isotype antibodies (rat IgG2b, mouse IgG2b, and mouse IgG1) corresponding to KSA7, 4M2B, 7M1, and 21M1 were each added to the TM7XN1-expressing cell line. Then the relationship between the expression levels of the TM7XN1-EGFP fusion protein and the binding amounts of the antibodies was studied. As revealed in Fig. 13, rat IgG2b (Fig. 13-1), mouse IgG2b (Fig. 13-2), and mouse IgG1 (Fig. 13-3) were each almost never bound, regardless of the expression levels of the TM7XN1-EGFP fusion protein.

Furthermore, KSA7, 4M2B, 7M1, and 21M1 were each separately added to the EGFP-expressing control cell line. Then, the relationship between the expression levels of EGFP and the binding amounts of the antibodies was studied. As revealed in Fig. 14, KSA7 (Fig. 14-1), 4M2B (Fig. 14-2), 7M1 (Fig. 14-3), and 21M1 (Fig. 14-4) were each almost never bound, regardless of the expression levels of EGFP.

As described above, it could be confirmed that KSA7, 4M2B, 7M1, and 21M1 had specifically bound to TM7XN1.

### Example 7: Competitiveness among anti-TM7XN1 monoclonal antibodies KSA7, 4M2B, 7M1, and 21M1 in terms of binding to TM7XN1

### Experimental materials

An HEK293 cell line forcibly expressing a TM7XN1-EGFP fusion protein was established at the Pharmaceutical Research Laboratories, Pharmaceutical Division, KIRIN Brewery Co., Ltd. and then cultured under conditions of DMEM + 10% FBS + 5 µg/mL Blasticidin, 37°C, and 5% CO₂. KSA7 is an anti-TM7XN1 monoclonal antibody (rat IgG2b) obtained by immunizing rats with a 3Y1 rat cell line forcibly expressing TM7XN1 at the Pharmaceutical Research Laboratories, Pharmaceutical Division, KIRIN Brewery Co., Ltd. KSA7 was prepared in the form of a 5 mg/mL PBS solution, stored, and then used as a purified antibody. Furthermore, KSA7 was labeled with Alexa Fluor 647 using Alexa Fluor 647 Protein Labeling Kit (Molecular Probes). Labeled KSA7 was prepared in the form of a 0.5 mg/mL PBS solution (supplemented with 0.05% (w/v) NaN₃) and then used as a labeled antibody. 4M2B, 7M1, and 21M1 are anti-TM7XN1 monoclonal antibodies (4M2B: mouse IgG2b; and 7M1 and 21M1: mouse IgG1) obtained via commissioned production by Immuno-Biological Laboratories Co., Ltd. Specifically, these antibodies were obtained by immunizing mice with a Balb3T3 mouse cell line forcibly expressing TM7XN1. 4M2B, 7M1, and 21M1 were each prepared in the form of a 2 mg/mL PBS solution, stored, and then used as a purified antibody. Furthermore, these antibodies were each labeled with Alexa Fluor 647 using an Alexa Fluor 647 Protein Labeling Kit (Molecular Probes), prepared in the form of a 0.5 mg/mL PBS solution (supplemented with 0.05% (w/v) NaN₃), and then used as a labeled antibody. FACSCalibur4A (Becton-Dickinson Japan) was used as a flowcytometer. A staining medium was prepared by adding 2% (v/v) FBS, 5 mM EDTA-2Na, and 0.05% (w/v) NaN₃ (all of these concentrations were final concentrations) to PBS. PI + staining medium was prepared by adding propidium iodide to the staining medium at 1 µg/mL (final concentration).

### Experimental methods

The HEK293 cell line forcibly expressing a TM7XN1-EGFP fusion protein (hereinafter, referred to as the TM7XN1-expressing cell line) was removed from a culture plate. The cells were prepared at a cell concentration of 1.0 × 10⁷ cells/mL using the staining medium. 25 µL each of the resultant was dispensed to a necessary number of 1.5-mL tubes. First, purified antibodies (KSA7, 4M2B, 7M1, and 21M1) were each added to a tube at 20 µg/mL (final concentration). 5 minutes later, labeled antibodies (KSA7, 4M2B, 7M1, and 21M1) were each further added to the tube at 0.2 µg/mL (final concentration). The above sample preparation was performed for a case of the labeled antibody alone and for all combinations of the purified antibodies and the labeled antibodies. After each resultant was allowed to stand at 4°C for 30 minutes, 1 mL of the staining medium was added. Centrifugation was performed at 400 G for 3 minutes to remove the supernatant and then the cells were washed. 350 µL of the PI + staining medium was added to each resultant so that the cells were suspended. The amount of each labeled antibody binding to the TM7XN1 molecule expressed on the surfaces of viable cells was measured using a flowcytometer and the Geo Mean values of fluorescence intensities.

### Results

Results of all the combinations of the labeled antibodies and the purified antibodies are listed as below using each labeled antibody as a standard.
(1) The results of the combinations of the KSA7 labeled antibody with the purified antibodies listed below are as shown in Fig. 15. Whereas the Geo Mean value in the case of the labeled antibody alone was 155.43 (Fig. 15-1), the Geo Mean value in the case in which KSA7 had been added (positive control) was 6.40 (Fig. 15-2), the Geo Mean value in the case in which 4M2B had been added was 156.18 (Fig. 15-3), the Geo Mean value in the case in which 7M1 had been added was 51.23 (Fig. 15-4), and the Geo Mean value in the case in which 21M1 had been added was 51.05 (Fig. 15-5). Hence, it can be said that KSA7 clearly competes with 7M1 and 21M1.
(2) The results of the combinations of the 4M2B labeled antibody with the purified antibodies listed below are as shown in Fig. 16. Whereas the Geo Mean value in the case of the labeled antibody alone was 156.25 (Fig. 16-1), the Geo Mean value in the case in which KSA7 had been added was 94.32 (Fig. 16-2), the Geo Mean value in the case in which 4M2B had been added (positive control) was 6.44 (Fig. 16-3), the Geo Mean value in the case in which 7M1 had been added was 134.96 (Fig. 16-4), and the Geo Mean value in the case in which 21M1 had been added was 131.15 (Fig. 16-5). Although 4M2B competed with KSA7, 7M1, and 21M1, the degrees of its competitiveness were low.
(3) The results of the combinations of the 7M1 labeled antibody with the purified antibodies listed below are as shown in Fig. 17. Whereas the Geo Mean value in the case of the labeled antibody alone was 204.85 (Fig. 17-1), the Geo Mean value in the case in which KSA7 had been added was 31.47 (Fig. 17-2), the Geo Mean value in the case in which 4M2B had been added was 216.75 (Fig. 17-3), the Geo Mean value in the case in which 7M1 had been added (positive control) was 8.78 (Fig. 17-4), and the Geo Mean value in the case in which 21M1 had been added was 8.61 (Fig. 17-5). Hence, it can be said that 7M1 clearly competes with KSA7 and 21M1.
(4) The results of the combinations of the 21M1 labeled antibody with the purified antibodies listed below are as shown in Fig. 18. Whereas the Geo Mean value in the case of the labeled antibody alone was 239.69 (Fig. 18-1), the Geo Mean value in the case in which KSA7 had been added was 33.43 (Fig. 18-2), the Geo Mean value in the case in which 4M2B had been added was 247.94 (Fig. 18-3), the Geo Mean value in the case in which 7M1 had been added (positive control) was 8.50 (Fig. 18-4), and the Geo Mean value in the case in which 21M1 had been added was 8.87 (Fig. 18-5). Hence, it can be said that 21M1 clearly competes with KSA7 and 7M1.

Based on the results of (1) to (4) above, the degrees of competitiveness of the combinations of all clones (in parentheses) were classified broadly based on ⊚: a high degree of competitiveness; ○: a medium degree of competitiveness; and Δ: a low degree of competitiveness. Classification results were (ΔKSA7, 4M2B), (○KSA7, 7M1), (○KSA7, 21M1), (Δ4M2B, 7M1), (Δ4M2B, 21M1), and (⊚7M1, 21M1). As described above, competitiveness was observed for all the 4 clones examined, but the degrees differed greatly from each other. It was considered that in the case of clones showing a high degree of competitiveness, their recognition sites may be common or located at positions extremely close to each other. It was considered that in the case of clones showing medium or low degrees of competitiveness, their recognition sites may be located close to each other, but the locations differ from each other. Therefore, it was considered that KSA7 and 4M2B are likely to differ from all clones other than themselves in terms of recognition site. It was also considered that 7M1 and 21M1 are likely to differ from KSA7 and 4M2B in terms of recognition site. In the meantime, as shown in the results in Example 3, all clones (KSA7, 4M2B, 7M1, and 21M1) were confirmed to possess significant activity of inhibiting bone marrow metastasis. Hence, even monoclonal antibodies that recognize different sites of the TM7XN 1 molecule can each possess the activity of inhibiting bone marrow metastasis. Therefore, it was demonstrated that the TM7XN 1 molecule is an important target molecule for suppression of metastasis of cancer cells.

All publications, patents, and patent applications cited herein are incorporated herein by reference in their entirety.

## Claims

1. A cancer-metastasis-suppressing agent, comprising an antibody against TM7XN1 as an active ingredient.

2. The cancer-metastasis-suppressing agent according to claim 1, wherein a cell of the cancer expresses TM7XN1 on its surface.

3. The cancer-metastasis-suppressing agent according to claim 1 or 2, wherein the cancer can metastasize to the bone.

4. The cancer-metastasis-suppressing agent according to claim 1 or 2, wherein the cancer can metastasize to the spleen.

5. The cancer-metastasis-suppressing agent according to any one of claims 1 to 4, wherein the cancer is selected from the group consisting of mammary cancer, prostate cancer, lung cancer, thyroid gland cancer, gastric cancer, malignant melanoma, neuroblastoma, multiple myeloma, acute myeloid leukemia, and pancreatic cancer.

6. The cancer-metastasis-suppressing agent according to any one of claims 1 to 5, being capable of suppressing the metastasis of a cancer cell to the bone marrow.

7. The cancer-metastasis-suppressing agent according to any one of claims 1 to 5, being capable of suppressing the metastasis of a cancer cell to the spleen.

8. A method for suppressing a cancer metastasis, comprising administering the cancer-metastasis-suppressing agent according to any one of claims 1 to 7 to a non-human animal with cancer.
